(19) **Europäisches Patentamt / European Patent Office / Office européen des brevets**

(11) **EP 3 579 844 B1**

(12) ## EUROPEAN PATENT SPECIFICATION

(45) Date of publication and mention
of the grant of the patent:
**11.03.2026 Bulletin 2026/11**

(21) Application number: **18750951.8**

(22) Date of filing: **12.02.2018**

(51) International Patent Classification (IPC):
| | |
|---|---|
| *A61K 35/35* (2015.01) | *A61K 35/28* (2015.01) |
| *A61P 7/00* (2006.01) | *A61P 17/02* (2006.01) |
| *A61P 37/04* (2006.01) | *A61P 19/00* (2006.01) |
| *C12N 5/0775* (2010.01) | *A61L 27/38* (2006.01) |
| *A61L 27/52* (2006.01) | *A61L 27/36* (2006.01) |
| *G01N 33/50* (2006.01) | *C12M 1/12* (2006.01) |
| *C12N 5/00* (2006.01) | *C12N 5/077* (2010.01) |

(52) Cooperative Patent Classification (CPC):
**A61K 35/35; A61K 35/28; A61L 27/3604;
A61L 27/3633; A61L 27/3886; A61L 27/3895;
A61L 27/52; A61P 7/00; A61P 17/02; A61P 19/00;
A61P 37/04; C12M 25/14; C12N 5/0068;
C12N 5/0653; C12N 5/0667;** (Cont.)

(86) International application number:
**PCT/US2018/017847**

(87) International publication number:
**WO 2018/148669 (16.08.2018 Gazette 2018/33)**

(54) **BIOLOGICAL SCAFFOLDS, PRODUCTS CONTAINING BIOLOGICAL SCAFFOLDS AND METHODS OF USING THE SAME**

BIOLOGISCHE GERÜSTE, PRODUKTE MIT BIOLOGISCHEN GERÜSTEN UND VERFAHREN ZUR VERWENDUNG DAVON

ÉCHAFAUDAGES BIOLOGIQUES, PRODUITS À ÉCHAFAUDAGES BIOLOGIQUES ET LEURS PROCÉDÉS D'UTILISATION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.02.2017 US 201762457366 P**

(43) Date of publication of application:
**18.12.2019 Bulletin 2019/51**

(73) Proprietor: **Obatala Sciences, Inc.
New Orleans, LA 70148 (US)**

(72) Inventors:
• **GIMBLE, Jeffrey**
**New Orleans, Louisiana 70112 (US)**
• **WU, Xiying**
**New Orleans, Louisiana 70112 (US)**
• **FRAZIER, Trivia**
**New Orleans, Louisiana 70112 (US)**

(74) Representative: **Cohausz & Florack
Patent- & Rechtsanwälte
Partnerschaftsgesellschaft mbB
Bleichstraße 14
40211 Düsseldorf (DE)**

(56) References cited:
| | |
|---|---|
| **EP-A1- 2 540 819** | **WO-A1-2007/103442** |
| **WO-A1-2014/145753** | **WO-A1-2017/207688** |
| **US-A- 5 904 716** | **US-A1- 2012 264 190** |
| **US-A1- 2016 143 955** | |

• **CHLAPANIDAS THEODORA ET AL: "Platelet lysate and adipose mesenchymal stromal cells on silk fibroin nonwoven mats for wound healing", JOURNAL OF APPLIED POLYMER SCIENCE, vol. 133, no. 5, 7 October 2015 (2015-10-07), US, pages n/a - n/a, XP055729730, ISSN: 0021-8995, DOI: 10.1002/app.42942**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• **XUANYE WU ET AL: "In situ characterization of the mTORC1 during adipogenesis of human adult stem cells on chip"**, PROCEEDINGS OF THE NATIONAL ACADEMY OF SCIENCES (PNAS), vol. 113, no. 29, 5 July 2016 (2016-07-05), pages E4143 - E4150, XP055729740, ISSN: 0027-8424, DOI: 10.1073/pnas.1601207113

(52) Cooperative Patent Classification (CPC): (Cont.)
**G01N 33/5014; G01N 33/5088;** C12N 2500/38; C12N 2501/33; C12N 2501/39; C12N 2501/999; C12N 2513/00; C12N 2533/54; C12N 2533/90

**Description**

CROSS REFERENCE TO RELATED APPLICATIONS

This European Patent claims the priority to and the benefit of United States Provisional Patent Application Serial No. 62/457,366 filed on February 10, 2017 and International Patent Application PCT/US2018/017847 filed on February 12, 2018.

BACKGROUND

The present disclosure relates to novel biological scaffolds which are derived from adipose tissue, products utilizing such scaffolds, and methods of use thereof.

While isolated human pre-adipocytes and adipocytes have been vastly utilized in two-dimensional (2D) cultures to identify signal transduction pathways relevant to healthy and diseased adipose tissue, the standard 2D culture model has substantial limitations. Two dimensional models fail to recapitulate the progression of adipocyte hyperplasia and hypertrophy characteristically associated with clinical obesity. Due to biomechanical constraints imposed by the 2D model, it is not possible to create the classic unilocular or "signet ring" adipocytes characteristic of human obesity. These unique structures are only possible within a three-dimensional (3D) model. Unlike the 2D model, the 3D tissue-engineered human SVF cell constructs will combine the biochemical, biomechanical, and biophysical signals that influence gene expression, cell polarity, and cell-cell interactions in a biomimetic environment. In contrast to the 3D model, the 2D monolayer model cannot trap the circulating hematopoietic cells that take up residence within native adipose tissue under both physiological and pathological conditions.

EP 2 540 819 A1 describes a method for cultivating cells 1 using a liquid medium 3 comprising blood platelet lysate and a gelatinization-inhibiting substance, said method comprising cultivating the cells 1 on the surface of and/or within a three-dimensional, gel-like substrate 4 comprising blood platelet lysate but being substantially devoid of a gelatinization-inhibiting substance, wherein the liquid medium 3 is layered on the gel-like substrate 4. Theodora et al., Journal of Applied Polymer Sciences, Volume 133, Issue 5, February 5, 2016, doi.org/10.1002/app.42942 describes platelet lysate (PL) and adipose-derived mesenchymal stromal cells (ASCs) seeded on nonwoven fibroin mats which were in vitro and in vivo evaluated for tissue regenerative applications.

WO 2014/145753 A1 describes a three dimensional cell culture and bioreactor system.

While certain novel features of this invention described below are pointed out in the annexed claims, the invention is not intended to be limited to the details specified, since a person of ordinary skill in the relevant art will understand that various omissions, modifications, substitutions and changes in the forms and details of the invention illustrated and in its operation, may be made.

BRIEF SUMMARY OF THE DISCLOSURE

The present disclosure provides a tissue-engineered, functional, humanized, cellular biological scaffold which permits the production of various biocompatible products for use in tissue engineering, tissue remodeling, and experimental models for the study of cell growth and differentiation and other drug discovery applications. The invention provides, a biological scaffold comprising a mammalian platelet lysate, optionally from human platelets, and stromal vascular fraction cells (SVF) comprising pre-adipocytes, vascular smooth muscle cells, endothelial cells and progenitors, B and T lymphocytes and progenitors, hematopoietic progenitors, macrophages, and monocytes, wherein said SVF cells are human cells. In some embodiments, the biological scaffold contains adipose-derived stromal cells and adipose derived stem cells.

In various embodiments, the biological scaffold exemplified herein has many applications for the study of cellular differentiation, creation of de novo tissues, for example, fat, cartilage, bone, blood cells, blood vessels, and combinations thereof. Moreover, the biological scaffold can be seeded by any cell type that requires a more natural physiological environment to simulate a disease process. Particular illustrative embodiments can include creation of patient derived xenograft tumors or other tumors from standard cancer cell lines which may be cultured in two-dimensional constructs or three-dimensional constructs in tissue culture or implanted in vivo, for example, in a mouse model.

Also disclosed herein are biological constructs which may be incorporated into products that may be further exploited.

Also described herein is an illustrative device incorporating the scaffolds disclosed herein and which may comprise a combination of a) one or more cellular components derived from an adipose tissue derived cellular fraction (ATDCF), a mammalian platelet lysate and optionally, a cell population, and b) a human-derived and/or silk-based, static, three-dimensional (3-D) culture with microfluidic form with inlets and outlets. The combination of cells and the biological scaffold enables short-term and extended 3-D in vitro culture of various combinations of one or multiple cell subtypes present within the ATDCF, or in vivo implantation into both small and large animal models. In certain embodiments, the present disclosure provides a tissue-engineered, humanized, adipose depot that includes the combination of an adipose tissue-resident stromal vascular fraction (SVF) cell population with a mammalian platelet lysate, wherein the platelet lysate may be a human platelet lysate. The cell/ biological scaffold combination enables short-term and extended three-dimensional in vitro culture of various combinations of one or multiple cell subtypes present within the SVF, or in vivo implantation into both small and large animal models. The silk, human platelet lysate, and ATDCF (SVF cells) allow for robust cell attachment, adipocyte differentiation, and the retention of the cellular diversity associated with both healthy and metabolically diseased human adipose depots. The constructs can be cultured for weeks or longer while maintaining their macroscopic and cellular structure and function. In addition, the biological scaffolds proposed herein are bioactive, biocompatible, free of donor DNA, human in origin, suitable for both autologous and allogeneic transplantation, and supportive of stromal/stem cell growth.

Both static and microfluidic *in vitro* constructs can be cultured for short-term or long-term culture while maintaining their macroscopic and cellular structure and function. In addition, the biological scaffolds proposed herein are bioactive, biocompatible, free of donor DNA, human in origin, suitable for both autologous and allogeneic transplantation, and supportive of stromal/stem cell growth.

In accordance with this discovery, it is an object of the disclosure to provide a static, microfluidic, 3-D in vitro platform for adipose tissue derived stem/stromal cells, stromal vascular fraction cells, or other primary cells that exist within adipose tissue.

It is an additional object of the disclosure to provide an *in vitro* humanized, or *in vivo* mouse, or other small or large animal-derived adipose tissue depot.

It is an additional object of the disclosure to provide methods of pharmacological drug testing and cancer microenvironment investigations.

Other objects and advantages of this disclosure will become readily apparent from the ensuing description.

BRIEF DESCRIPTION OF THE DRAWINGS

The following drawings form part of the present specification and are included to further demonstrate certain aspects of the present disclosure. The disclosure may be better understood by reference to one or more of these drawings in combination with the description of specific embodiments presented herein.

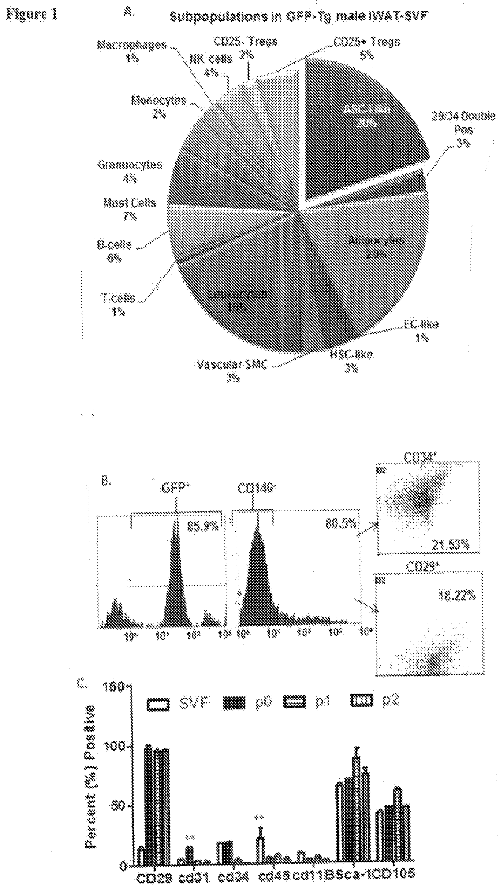

Reference Figure 1 shows the characterization of cells used in the study. 1a). Pie chart of subpopulations within GFP-Tg SVF cells that were utilized for SVF serial transplantation studies. 1b). Sorting and enrichment of CD146- CD29+, and CD146- CD34+ subpopulations that were culture expanded to P2 ASC for GFP-Tg ASC serial transplantation studies. 1c). Immunophenotype of GFP-Tg SVF cells and culture-expanded GFP-Tg ASC, until passage 2 (P2). All experiments were repeated in triplicate; sample size, n=3 per replicate. Values reported as mean + standard deviation ($\mu$ + SD); *p < 0.05, ** p < 0.01; ***p < 0.001.

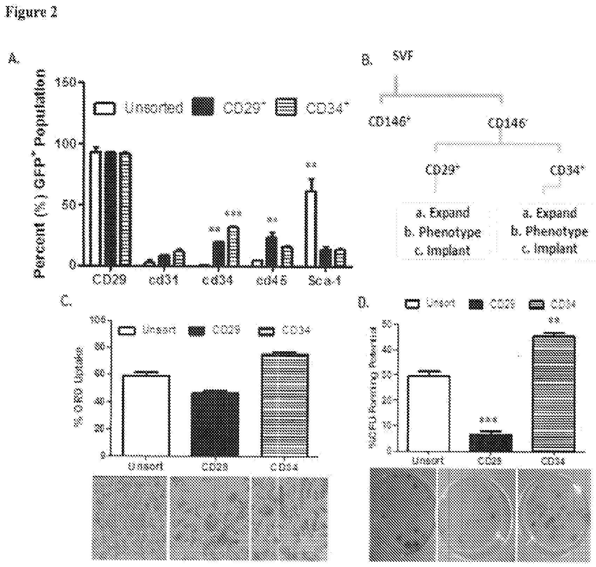

Reference Figure 2 shows the comparison of CD29-enriched and CD34-enriched GFP-Tg ASC. 2a). Immunophenotype of CD146- CD29+, and CD146- CD34+ sorted GFP-Tg ASC subpopulations. Immunophenotypes were based on expression of CD29, CD31, CD34, CD45, CD11B, Sca-1, and CD105 surface antigens. 2c). Adipogenic differentiation, and 2d) colony formation assays of unfractionated GFP-Tg ASC, CD146- CD29+ GFP-Tg ASC, and CD146-CD34+ GFP-Tg ASC. All experiments were repeated in triplicate; sample size, n=3 per replicate. Values reported as mean + standard deviation ($\mu$ + SD); *p < 0.05, ** p < 0.01; ***p < 0.001.

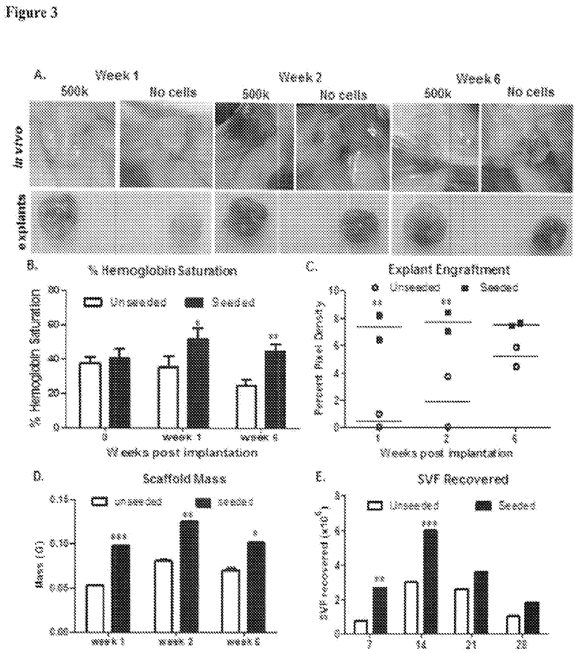

Reference Figure 3 shows weekly progression of tissue engineered fat within and surrounding GFP-Tg SVF cell implants in mice. 3a). Scaffolds were implanted with no cells, or with 500k GFP-Tg SVF cells in silk scaffolds. Scaffolds were removed following 1week, 2 weeks, or 6 weeks of implantation. 3b) Percent hemoglobin saturation was measured immediately following implantation, and after 1 week and 6 weeks of initial implantation. 3c). Quantification of removed scaffold (explant) engraftment via ImageJ analyses of photomicrographs in 3a; sample size, n=6 per group. 3d). Scaffold mass measurements following weeks 1, 2, and 6 of implantation; sample size, n=20. Quantification supports photomicrographs of SVF-mediated engraftment acceleration after 1 week of implantation. 3e). Weekly SVF cell quantification correlate with SVF cell persistence and expansion by week 2, and differentiation. Values reported as mean + standard deviation ($\mu$ + SD); *p < 0.05, ** p < 0.01; ***p < 0.001.

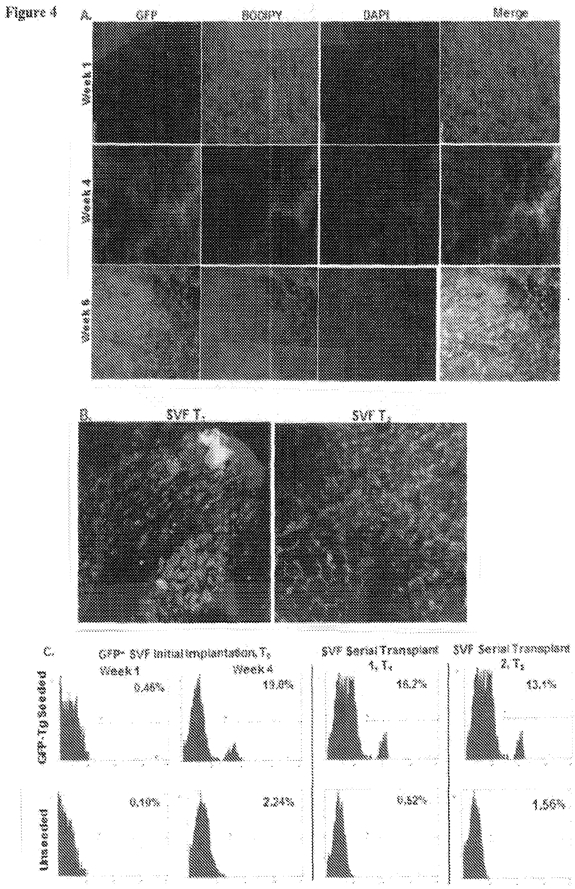

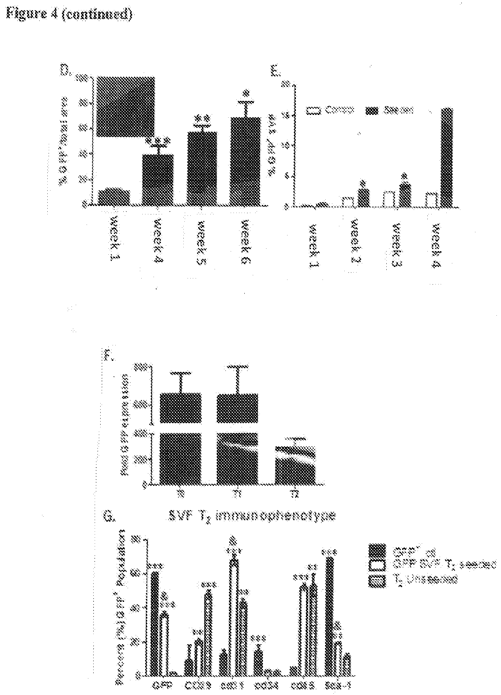

Reference Figure 4 shows detection of GFP-Tg SVF from tissue engineered adipose using HFIP 6-week silk scaffold implants in mice over two serial transplantations. Photomicrographs of GFP-Tg SVF cell implantation following weeks 1, 4, and 6 in vivo demonstrate persistence, proliferation, and ability to form GFP-Tg adipose depots. 4a). Confocal microscopy images of GFP, BODIPY, DAPI, and merged images within initial 6-week GFP-Tg SVF cell (T0) transplants. 4b) Merged GFP/BODIPY/DAPI images of removed 6-week GFP-Tg SVF cell first serial (T1) and second serial (T2) transplants; sample size, n=5. 4c) Flow cytometric analyses of GFP expression in removed adipose scaffolds following T0, T1, and T2 transplants; sample size, n=18. 4d). ImageJ analysis of weekly confocal images of T0 explants. 4e) Quantification of weekly flow cytometric analyses of GFP expression in removed T0 GFP-Tg SVF cell implants. 4f). GFP DNA expression in samples from removed 6-week T0, T1, and T2 transplants. GFP expression reported as fold expression and normalized to GAPDH expression. 4g). Immunophenotype based on expression of GFP, CD29, CD31, CD34, CD45, and Sca-1 antigen expression on SVF cells isolated from 6-week T2 transplants. Values reported as mean + standard deviation ($\mu$ + SD); comparing individual grouping to GFP+ control: *$p < 0.05$, ** $p < 0.01$; ***$p < 0.001$; comparing to GFP-SVF T2 seeded to unseeded control groups: & $p < 0.05$.

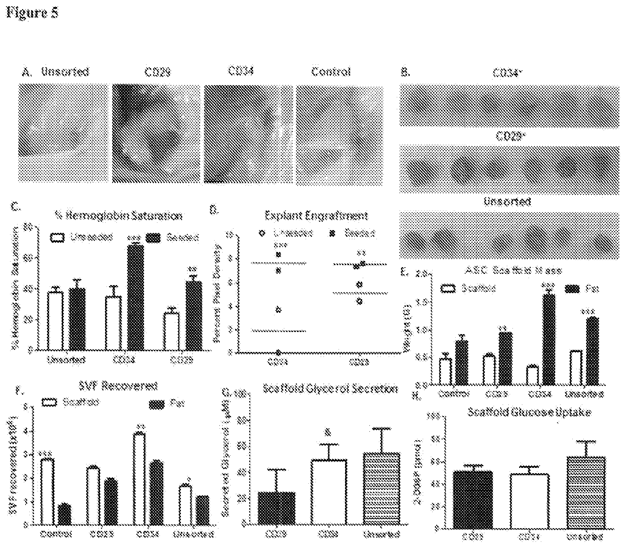

Reference Figure 5 shows adipose formation and functionality within GFP-Tg ASC seeded serial silk implants. 5a). Photomicrographs of scaffolds that were implanted with no cells (control), or with (a), 500k unsorted GFP-Tg ASC; (b), CD146- CD29+ GFP-Tg ASC; or (c), CD146- CD34+ GFP-Tg ASC; in silk scaffolds. Scaffolds were removed following 6 weeks of implantation. 5b). Images of removed scaffolds from cohorts (a)-(c). 5c) Percent hemoglobin saturation was measured in groups (a)-(c) after 6 weeks of initial (T0) implantation. 5d). Quantification of removed scaffold (explant) engraftment via ImageJ analyses of photomicrographs in 4b; sample size, n=6 per grouping. 5e). Scaffold mass measurements after 6-week implants of cohorts (a)-(c), and control implants that were not seeded with any cells. 5f). Quantification of SVF recovered from 6-week implants, and surrounding fat from cohorts (a)-(c), and the unseeded control group; sample size, n=18 per grouping. Functionality was measured via 5g) glucose uptake, and 5f) glycerol secretion assays using 6-week T1 transplantation constructs. Data analyzed using Graphpad Prism. Two-way ANOVAs performed. Data reported as mean + SD. *$p < 0.05$, ** $p < 0.01$; ***$p < 0.001$; comparing to CD34-enriched group: & $p < 0.05$.

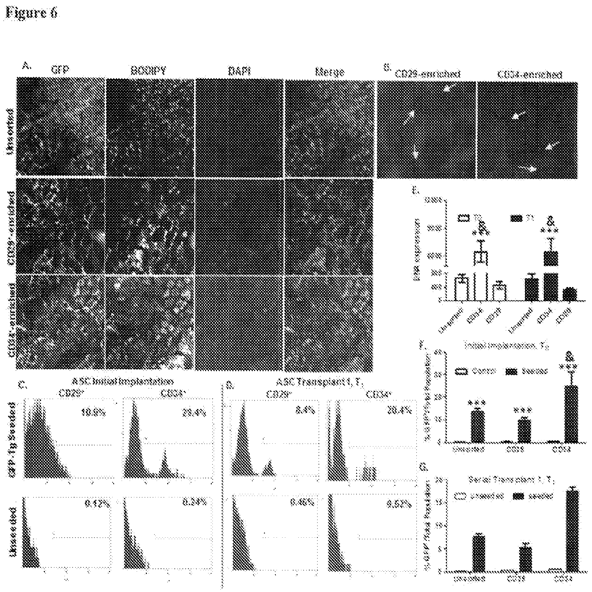

Reference Figure 6 shows detection of CD146- CD29+ GFP-Tg ASC and CD146-CD34+ GFP-Tg ASC from tissue engineered adipose using HFIP 6-week silk scaffold implants in mice over two serial transplantations. Photomicrographs of GFP-Tg CD29-enriched and CD34-enriched (cohorts (a)-(c) cell implantation following week 6 in vivo demonstrate persistence, proliferation, and ability to form GFP-Tg adipose depots, similar to unsorted GFP-Tg SVF cohorts. 6a). Confocal microscopy images of GFP, BODIPY, DAPI, and merged images from 6-week GFP-Tg ASC serial (T1) transplants. 6b) Confocal images of CD29-enriched and CD34-enriched 6-week T1 groups reflected observation of micro vessel formation within CD34-enriched groups than in CD29-enriched groups; n=5 per group per replicate. Flow cytometric analyses of GFP expression in removed adipose scaffolds from cohorts (a)-(c) following 6c) T0, and 6d) T1 transplants. 6e). GFP DNA expression in samples from removed 6-week T0 and T1 transplants. GFP expression reported as fold expression and normalized to GAPDH expression. Quantification of %GFP positivity following 6-week transplantation in 6f) T0 and 6g) T1 GFP-Tg ASC cell implants. Data reported as mean + SD. Comparing individual grouping to unsorted cell seeded groups: *$p < 0.05$, ** $p < 0.01$; ***$p < 0.001$; comparing to CD34-enriched group: & $p < 0.05$.

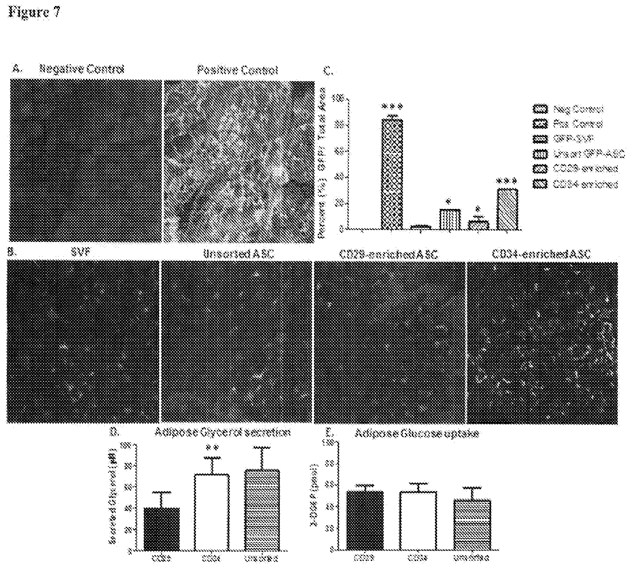

Reference Figure 7 shows GFP-Tg SVF cells, CD29-enriched, and CD34-enriched GFP-Tg ASC infiltrate surrounding tissue to generate functional, GFP-Tg adipose. Confocal microscopic images of fat within 2mm surrounding SVF and ASC constructs revealed GFP positivity. 7a) Merged images of negative and positive control adipose stained with BODIPY and DAPI in non-GFP-Tg mice and GFP-Tg mice, respectively. 7b) Images of adipose surrounding 6-week constructs that were seeded with unsorted GFP-Tg SVF cells, unsorted GFP-Tg ASC, and CD146- CD29-enriched and CD34-enriched GFP-Tg ASC; n=5 per group per replicate. 7c) Quantification of confocal images from 7a and 7b. Functionality reported via 7d) glycerol secretion, and 7e) glucose uptake assays using 6-week T1 transplantation constructs. Data reported as mean + SD. *$p < 0.05$, ** $p < 0.01$; ***$p < 0.001$.

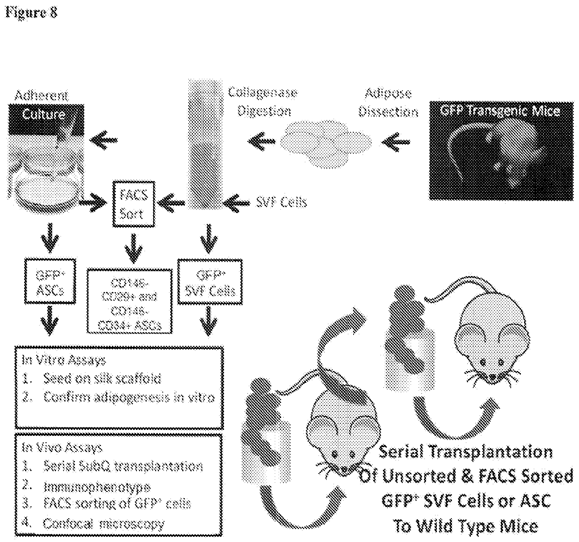

Reference Figure 8 shows the overall process involved in the development of the silk-based, 3-D culture. SVF cells were isolated from inguinal white adipose tissue of GFP-Tg C57Bl/6 mice according to established protocols (see materials and methods). CD146- GFP-Tg SVF cells were sorted and selected based on CD29+ and CD34+ expression. CD29+-enriched, CD34+-enriched, and unsorted cells were either seeded on silk scaffolds and implanted into non GFP-Tg mice, or ASC-like populations were culture expanded to passage 2, seeded on silk scaffolds, and implanted in non-Tg mice. Following 6 weeks of implantation, engineered tissue constructs were removed, digested using collagenase tissue digestion protocols (see materials and methods). Isolated cells were plated, expanded, and serially transplanted.

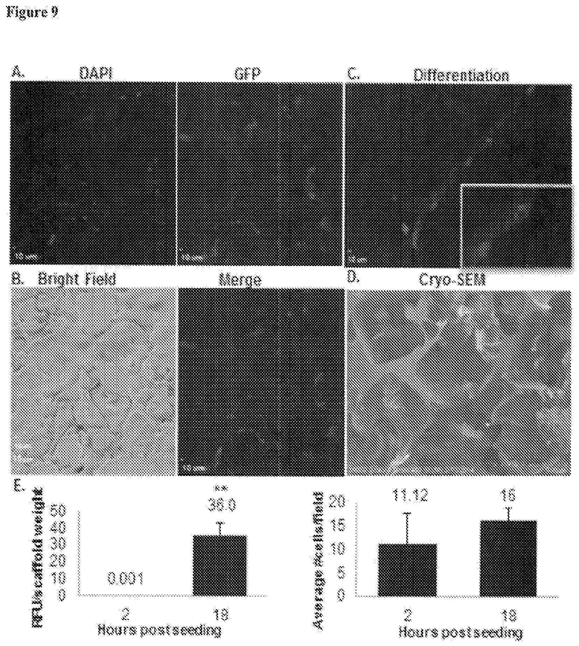

Reference Figure 9 shows in vitro seeding of GFP-Tg ASC on silk scaffolds for implantation studies. 9a). Confocal x100 magnification images of DAPI and GFP expression, 9b) Bright field and merge of DAPI/GFP expression in metabolically active GFP-Tg ASC 18hrs post seeding on silk scaffolds in vitro. 9c). Confocal microscopy image of adipogenic differentiated GFP-Tg ASC 5 days in adipogenic medium. 9d). cryogenic scanning electron microscopy (cryo-SEM) image of unseeded scaffolds at x80 magnification. Metabolically active GFP-Tg ASC that are present 9e), via Alamar Blue staining, and adhered 9f), via DAPI staining 2 and 18 hours after loading onto silk scaffolds in vitro. Relative Fluorescence Intensity (RFU) as determined by Alamar Blue analysis was normalized to scaffold wet weight and used as a measure of cell attachment levels 2 hr. and 18hr post ASC seeding within each scaffold group. All experiments were repeated in triplicate; sample size, n=5 per replicate. Values reported as mean + standard deviation ($\mu$ + SD); ** $p < 0.01$.

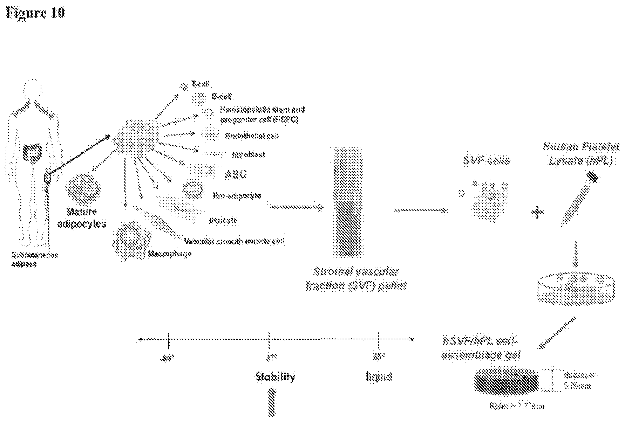

Figure 10 shows a schematic of the process for assemblage of the thermo-responsive 3-D cultures of human stromal vascular fraction and human platelet lysate (SVF/haply 3-D constructs). Human SVF cells are isolated from digested human adipose tissue. hSVF cells are combined with optimal concentrations of hPL supplemented stromal media, and incubated in 37°C with 5% $CO_2$ infusion. The thermos-responsive self-assembling matrices are formed immediately upon mixing the cell-hPL/media combination.

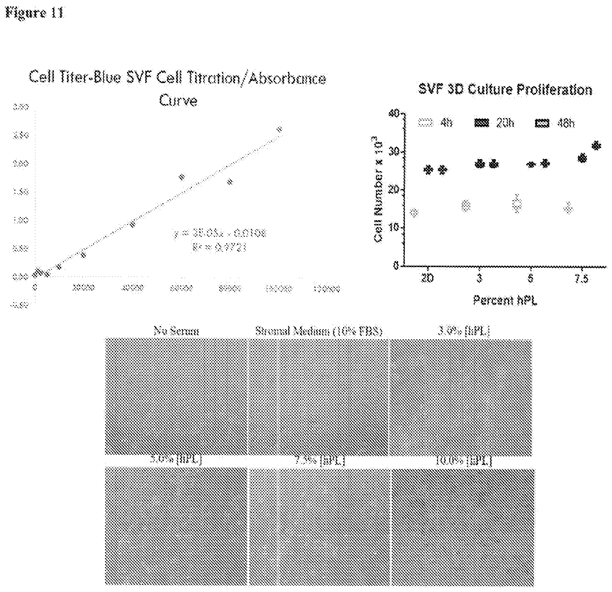

Figure 11 shows the proliferative potential of human SVF cells cultured in the 3-D conditions in hPL compared to traditional 2-D monolayer cultures. Cell proliferation was measured via Cell Titer-Blue reagent after culture for 3, 5 and 7 days in 2-D or 3-D culture conditions. Metabolically active SVF that are present and adhered are quantified 24, 48, and 72 hours after loading onto silk scaffolds in vitro. Cell number as determined by Cell Titer-Blue absorbance analysis was normalized to cell number titration standard curves and used as a measure of cell growth within each hPL matrix group. All experiments were repeated in triplicate; sample size, n=3 per replicate. Values reported as mean + standard deviation ($\mu$ + SD); * p < 0.05.

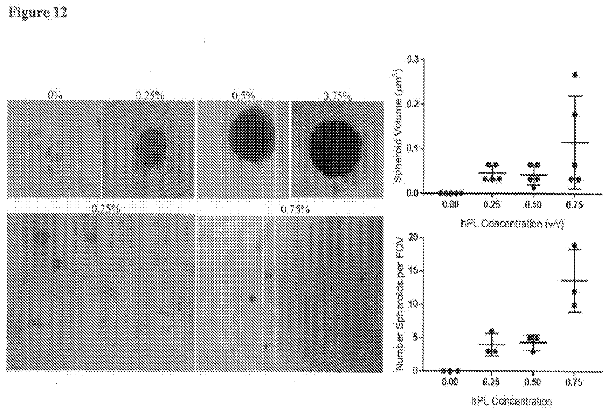

Figure 12 shows hSVF spheroid formation in 3D hPL cultures. Photomicrographs demonstrate that spheroid formation occurs at low concentration hPL. Spheroid size and density are both directly correlated with percent hPL concentration in stromal media. All experiments were repeated in triplicate; sample size, n=3 per replicate. Values reported as mean + standard deviation ($\mu$ + SD); * p < 0.05.

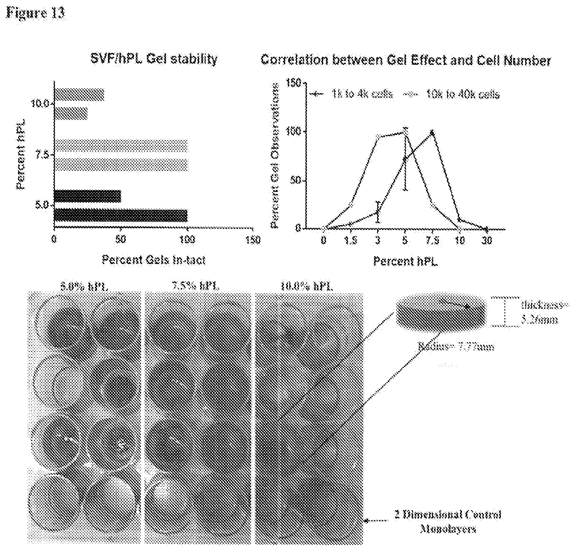

Figure 13 shows the correlation between cell-mediated matrix gelling and matrix stability in relation to cell number. All experiments were repeated in triplicate; sample size, n=5 per replicate. Values reported as mean + standard deviation ($\mu$ + SD); * p < 0.05.

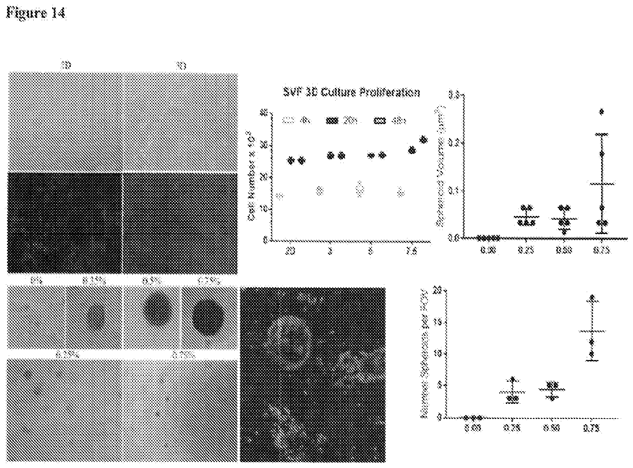

Figure 14 shows the proliferative and spheroid-forming behavior of stromal vascular fraction when placed in culture with hPL-based matrices. Cell proliferation was measured via Cell Titer-Blue reagent after culture for 3, 5 and 7 days in 2-D or 3-D culture conditions. Metabolically active human SVF that are present and adhered are quantified 24, 48, and 72 hours after seeding with the matrices. Cell number as determined by Cell Titer-Blue absorbance analysis was normalized to cell number titration standard curves and used as a measure of cell growth within each hPL matrix group. Cell proliferation and spheroid formative per field of view (FOV) was determined by phase contrast or confocal LASER microscopy stained with Hoescht (nuclei) or Bodipy (lipid vacuole). Spheroid size and density are both directly correlated with percent hPL concentration in stromal media. Photomicrographs demonstrate that spheroid formation occurs at low concentration hPL. All experiments were repeated in triplicate; sample size, n=5 per replicate. Values reported as mean $\pm$ standard deviation ($\mu \pm$ SD); * p < 0.05.

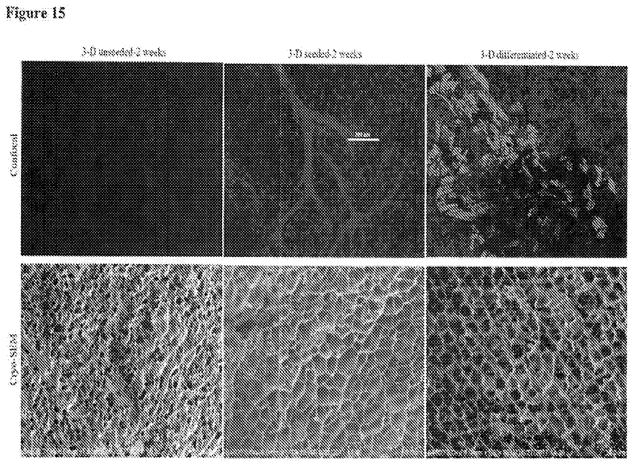

Figure 15 depicts photomicrographs of unseeded and human SVF cells seeded on human platelet lysate scaffolds. Confocal images stained with Hoescht and Bodipy dyes were merged for 3-D human platelet lysate cultures maintained for 3 weeks in vitro in without or with adipogenic differentiation medium. Identical cultures were fixed for cryogenic scanning electron microscopy (cryo-SEM), and imaged at 30-50$\mu$m magnifications. All experiments were repeated in triplicate; sample size, n=3 per replicate.

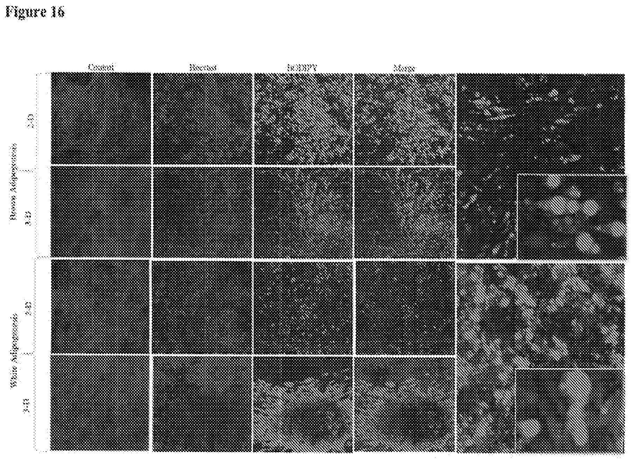

Figure 16 depicts the in vitro formation of human white adipose tissue (hWAT) and beige/brite adipose tissue (hbAT) using SVF cells and hPL-based matrices. SVF cells were seeded with hPL matrices and induced to undergo adipogenesis using a white or beige/brite adipogenic cocktail for two weeks in culture. Confocal images (100x magnification) stained with Hoescht and Bodipy dyes were merged for 3-D human platelet lysate cultures. All experiments were repeated in triplicate; sample size, n=3 per replicate.

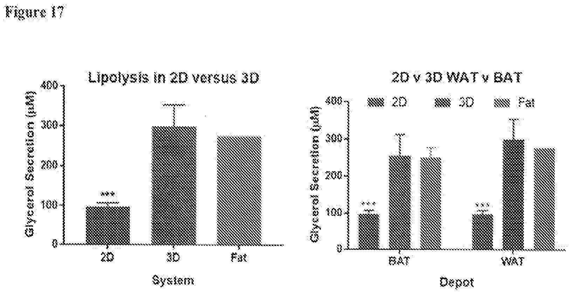

Figure 17 shows quantification of human white adipose tissue (hWAT) and beige/brite adipose tissue (hbAT) formed in culture using SVF cells and hPL-based matrices. SVF cells were seeded with hPL matrices and induced to undergo adipogenesis using a white or beige/brite adipogenic cocktail for two weeks in culture. Confocal images (100x magnification) of SVF/hPL-matrix constructs and positive control human fat were stained with Hoescht and Bodipy dyes. Confocal images were quantified using Cell Profiler software (www.cellprofiler.org). All experiments were repeated in triplicate; sample size, n=5 per replicate. Values reported as mean $\pm$ standard deviation ($\mu \pm$ SD); * p < 0.05.

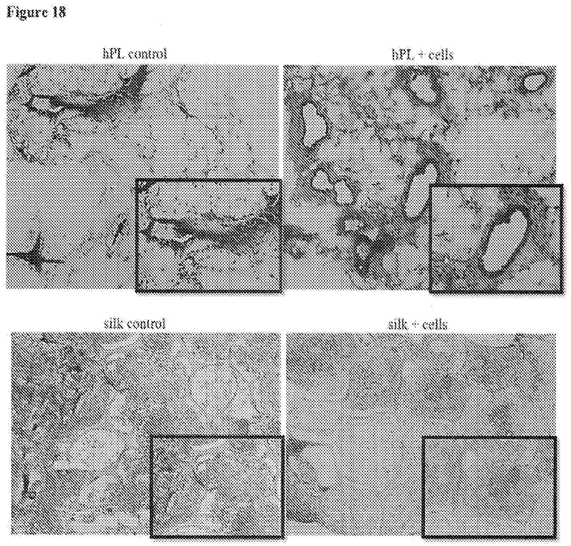

Figure 18 shows in vivo implant of hSVF Cell/human platelet lysate bioscaffold in Nude Mice. Subcutaneous implants were harvested after 8 weeks, fixed in 4% paraformaldehyde, and sectioned in 10$\mu$m slices. Sections were mounted on imaging slides, stained for nuclei (Hematoxylin) or neutral lipids (Eosin), and visualized by slide scanner. All experiments were repeated in triplicate; sample size, n=3 per replicate.

DETAILED DESCRIPTION

Detailed descriptions of one or more preferred embodiments are provided herein. It is to be understood, however, that the present disclosure may be embodied in various forms. Therefore, specific details disclosed herein are not to be interpreted as limiting, but rather as a basis for the claims and as a representative basis for teaching one skilled in the art to employ the present disclosure in any appropriate manner.

Wherever any of the phrases "for example," "such as," "including" and the like are used herein, the phrase "and without limitation" is understood to follow unless explicitly stated otherwise. Similarly, "an example," "exemplary" and the like are understood to be non-limiting.

The term "substantially" allows for deviations from the descriptor that do not negatively impact the intended purpose. Descriptive terms are understood to be modified by the term "substantially" even if the word "substantially" is not explicitly recited. Therefore, for example, the phrase "wherein the lever extends vertically" means "wherein the lever extends substantially vertically" so long as a precise vertical arrangement is not necessary for the lever to perform its function.

The terms "comprising" and "including" and "having" and "involving" (and similarly "comprises", "includes," "has," and "involves") and the like are used interchangeably and have the same meaning. Specifically, each of the terms is defined consistent with the common United States patent law definition of "comprising" and is therefore interpreted to be an open term meaning "at least the following," and is also interpreted not to exclude additional features, limitations, aspects, etc. Thus, for example, "a process involving steps a, b, and c" means that the process includes at least steps a, b and c. Wherever the terms "a" or "an" are used, "one or more" is understood, unless such interpretation is nonsensical in context.

The term "depot" means a specific site on the body in which a tissue (e.g., adipose) is commonly found.

The term "pharmacological agent" whether used as a component of a biological scaffold, or for any of the methods for using a biological scaffold, has a meaning known to those of skill in the art, and will include at least the following examples of active agents: growth factors, differentiation factors, cytokines, chemokines, adipokines, antibiotics, antimycotics, anti-fungals, anti-inflammatories, and anti-cancer agents (chemotherapeutic agents). The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

The present invention provides a biological scaffold comprising a mammalian platelet lysate, optionally from human platelets, and stromal vascular fraction cells (SVF) comprising pre-adipocytes, vascular smooth muscle cells, endothelial cells and progenitors, B and T lymphocytes and progenitors, hematopoietic progenitors, macrophages, and monocytes, wherein said SVF cells are human cells. In some embodiments, the biological scaffold contains adipose-derived stromal cells and adipose derived stem cells. Adipose-derived stromal cells and adipose derived stem cells are collectively referred to herein as "ASC". Described herein are also bone marrow-derived mesenchymal stromal cells and bone marrow-derived mesenchymal stem cells which are collectively referred to herein as BM-MSC.

As used herein the term "Stromal Vascular Fraction (SVF) Cells" includes within its definition a heterogeneous cell population isolated from adipose tissue following enzymatic or mechanical disruption/digestion with an exemplary protease, for example, collagenase type 1 or other related enzyme capable of disruption the extracellular matrix. The SVF cells have not adhered to plastic or undergone expansion/proliferation in vitro. This population contains pre-adipocytes, stromal/stem cells, lymphoid cells, myeloid cells, fibroblasts, pericytes, endothelial progenitors, and hematopoietic stem cells, among others.

Adipose-derived Stromal/Stem Cells (ASC): The relatively homogeneous cell population isolated from adipose SVF cells following adherence to plastic tissue plates and culture expansion in the presence of growth factors/medium in vitro. This population is relatively enriched in adherent pre-adipocytes, stromal/stem cells, fibroblasts, pericytes, and endothelial progenitors with a depletion of the lympho-hematopoietic lineages. The ASC are multipotent, being capable of adipocyte, chondrocyte, and osteoblast differentiation.

Bone Marrow-derived Mesenchymal Stromal/Stem Cells (BM-MSC): The relatively homogeneous cell population isolated from bone marrow aspirates following adherence to plastic tissue plates and culture expansion in the presence of growth factors/medium in vitro. The BM-MSC are similar in phenotype to ASC with multipotent differentiation potential (adipocyte, chondrocyte, osteoblast).

## A. Biological Scaffolds

In one embodiment, the present disclosure provides a biological scaffold that may be augmented with a variety of cell types, and a variety of growth factors, cellular differentiation factors and other agents useful in the study of cell growth, for example, inhibitors of tumor or cancer growth. According to the invention, the biological scaffold contains at least two components. The first component is a mammalian platelet lysate, optionally from human platelets. Also described herein are "mammalian platelet lysates" which can include a lysate formed from platelets isolated from any mammalian source.

The mammalian platelet lysate described herein may include a platelet lysate made from platelets of any natural mammalian platelet or cultured platelet. According to the invention, a mammalian platelet lysate may include a human platelet lysate as used herein in the various products and uses of the biological scaffold described herein. According to the invention, the platelets used in the manufacture of the mammalian platelet lysate for use in the biological scaffolds disclosed in the compositions, products and methods of use are human platelets. The mammalian platelet lysate can be created in a liquid format that can be cryopreserved as a frozen material to be thawed immediately prior to use; however, the product can also be processed and stored using alternative methods and physical formats including but not limited to lyophilized, particulated, solidified, or as a combination product (as outlined below). According to the invention, a form of a mammalian platelet lysate may include a liquid product derived from human platelet lysates. Sterile, expired human platelets collected by an AABB licensed blood collection center are received frozen at -20°C for processing in the laboratory. All steps where the platelets are exposed to air are conducted within a biological safety cabinet (BSL2). The platelets are subjected to three consecutive freeze/thaw cycles alternating between room temperature (15°C to 25°C) and -80°C. Following the final thaw cycle, the platelets from one to fifty donors are pooled and centrifuged at 8,000 X g (rcf). Following the centrifugation step, aspirate the top white layer containing the platelet membranes. The remaining clear infranatant, corresponding to the platelet lysate, comprises the mammalian platelet lysate product in its undiluted state.

The second component of the biological scaffolds of the invention are stromal vascular fraction cells (SVF) comprising pre-adipocytes, vascular smooth muscle cells, endothelial cells and progenitors, B and T lymphocytes and progenitors, hematopoietic progenitors, macrophages, and monocytes. Also described herein is an adipose tissue derived cellular fraction (ATDCF). This term refers to at least one cell type which is derived from adipose tissue. In some embodiments, the biological scaffold of the present disclosure contains adipose-derived stromal cells and adipose derived stem cells, (collectively referred to herein as "ASC"). The ATDCF described herein comprises at least one of stromal vascular fraction (SVF) cells, adipose-derived stromal cells, adipose derived stem cells, bone marrow-derived mesenchymal stromal cells and bone marrow-derived mesenchymal stem cells and the ATDCF is derived from adipose tissue. The biological scaffolds of the present disclosure may be tailored for specific uses by complementing the mammalian platelet lysate with one or more of these cell types, optionally with one or more pharmacological agents (for example, growth factors, differentiation factors, cytokines, chemokines, adipokines, antibiotics, antimycotics, anti-fungals, anti-inflammatories, anti-cancer agents (chemotherapeutic agents etc.) to provide a biological scaffold containing product, a differentiation construct, a graft, a wound dressing and other applications disclosed herein.

## B. Products Incorporating the Biological Scaffold

### Kits For Cellular Differentiation.

The biological scaffolds described herein may be used as starting components for practitioners to develop specific differentiated cells and tissues. In some illustrative examples, these kits may be used for research purposes. For example, an illustrative kit may contain a container supplemented with a mammalian platelet lysate, a multi-well substrate, and reagent A (consists of Dulbecco's Modified Essential Medium, supplemented with 1% antibiotic/antimycotic, and other related cell culture medium containing necessary nutrients, amino acids, and growth factors for cell culture and white or brown adipogenic differentiation). In specific examples, in which the user may wish to prepare differentiated cells in a 3-dimension (3-D) cell culture the 3-D tissue culture using may be prepared in accordance with the following illustrative steps: Prepare a mammalian platelet lysate supplemented media by adding 25% (v/v) of the mammalian platelet lysate to 1% antibiotic/antimycotic and 74% Dulbecco's Modified Essential Medium or other related cell culture medium containing necessary nutrients, amino acids, and growth factors for cell culture. After mixing, do not warm in water bath. Maintain at room temperature. Thaw cells (SVF cells, and/or ASCs and/or BM-MSCs) from liquid nitrogen in 37°C water bath until the moment ice disappears. Rinse each cryovial once with 1mL standard Stromal Medium. Centrifuge tube at 300x g (1200 rpm) for 5 minutes. Re-suspended the cell pellet in hPL supplemented Medium at a concentration of $5x10^5$ cells per mL. Immediately plate the cell/hPL mixture. Mixture will immediately gel when placed in $CO_2$ incubator. Maintain the 3-D culture by replacing evaporated culture media every 7 days. Remove the old media in the cultures by aspiration via a 1mL micropipette. Replace with fresh media by adding a half-volume amount of the hPL-media to the top corner of the gel.

In another related example, differentiated adipose cells may be induced in a biological scaffold of the present disclosure. In an illustrative method, a user may utilize the mammalian platelet lysate, a substrate for culturing white or brown adipose cells/tissue, and reagent A. Prepare the 3-D tissue culture using the following instructions: Prepare mammalian platelet lysate supplemented media by adding 25% (v/v) mammalian platelet lysate to 1% antibiotic/antimycotic and 74% Dulbecco's Modified Essential Medium or other related cell culture medium containing necessary nutrients, amino acids, and growth factors for cell culture. After mixing, do not warm in water bath. Maintain at room temperature. Thaw stromal vascular fraction (SVF), bone marrow-derived mesenchymal stem/stromal (BM-MSC), or adipose stromal/stem (ASC) cells provided in cryovials from liquid nitrogen in 37°C water bath until the moment ice disappears. Rinse each cryovial once with 1mL standard Stromal Medium. Centrifuge tube at 300x g (1200 rpm) for 5 minutes. Re-suspended the cell pellet in hPL supplemented Medium at a concentration of $5x10^5$ cells per mL. Immediately plate the cell/hPL mixture. Mixture will immediately gel when placed in $CO_2$ incubator. Maintain the 3-D culture by replacing evaporated culture media every 7 days. Remove the old media in the cultures by aspiration via a 1mL micropipette. Replace with fresh media by adding a half-volume amount of the hPL-media to the top corner of the gel.

In a related example, a kit for the differentiation of precursor cells to adipose tissue, or brown/beige adipose tissue is provided. In an exemplary kit, the kit may contain a mammalian platelet lysate in the form of a liquid product, which may be combined with one or more cryovials of SVF cells derived from adipose tissue, one or more 100 ml bottles of medium of LaCell LLC (LaCell LLC, New Orleans, Louisiana, USA) products specific for cell expansion (StromaQual), adipogenic differentiation (AdipoQual), chondrogenic differentiation (ChondroQual), or osteoblastic differentiation (OsteoQual), and tissue culture plates (6, 12, or 24 well) suitable for sterile cell growth and differentiation. The kit may be further modified by inclusion of proprietary adipogenic differentiation media designed to promote selectively white adipose tissue (or brown/beige adipose tissue.

In a related example, a kit for cell expansion (StromaQual), adipogenic differentiation (AdipoQual), chondrogenic differentiation (ChondroQual), or osteoblastic differentiation (OsteoQual) is provided. In an exemplary kit, the kit may contain a frozen tube of the mammalian platelet lysate in the form of a liquid product, one or more cryovials of ASC derived from adipose tissue, one or more 100 ml bottles of medium of LaCell LLC products specific for cell expansion (StromaQual), adipogenic differentiation (AdipoQual), chondrogenic differentiation (ChondroQual), or osteoblastic differentiation (OsteoQual), and tissue culture plates (6, 12, or 24 well) suitable for sterile cell growth and differentiation. The kit may be further modified by inclusion of proprietary adipogenic differentiation media designed to promote selectively white adipose tissue or brown/beige adipose tissue.

In a related example, a kit for cell expansion (StromaQual), adipogenic differentiation (AdipoQual), chondrogenic differentiation (ChondroQual), or osteoblastic differentiation (OsteoQual) is provided. In an exemplary kit, the kit may contain a frozen tube of the mammalian platelet lysate in the form of a liquid product, one or more cryovials of BM-MSC derived from bone marrow aspirates, one or more bottles of medium of LaCell LLC products specific for cell expansion (StromaQual), adipogenic differentiation (AdipoQual), chondrogenic differentiation (ChondroQual), or osteoblastic differentiation (OsteoQual), and tissue culture plates (6, 12, or 24 well) suitable for sterile cell growth and differentiation. The kits described herein may be further modified by inclusion of proprietary adipogenic differentiation media designed to promote selectively white adipose lineage or brown/beige adipose lineage differentiation.

Also described herein is an illustrative biological scaffold that may be used without modification for tissue reconstruction, or cosmetic purposes, or may be applied to a solid or semi-solid surface for incorporation into or adjacent a tissue defect, for example, a wound or damaged bone. The biological scaffold may include a solution of mammalian platelet lysate, for example, a human platelet lysate, prepared using the methods described herein mixed directly with SVF cells derived from adipose tissue as a combination product that can be used as an injectable material or processed as a solid or semi-solid scaffold which may be implantable into the tissue defect, for example, bone for tissue repair. In some embodiments, the biological scaffold may be inserted or applied to at least one surface of an orthopedic device, for example, bone cages, bone screws, bone pedicles and other orthopedic devices which have at least some contact with the patient's bone. The sections or portions of diseased bone may be treated using the biological scaffold or solid substrates containing or coated with an exemplary biological scaffold described herein. In non-limiting examples, osteonecrosis of the jaw or the femoral head may be treated with resection of the diseased bone tissue followed by implantation of transplanted autologous or allogeneic bone tissue in which at least one surface of the transplanted bone is coated or contains an exemplary biological scaffold, for example, a biological scaffold containing human platelet lysate and BM-DSCs. In an exemplary osteonecrosis repair of the femoral head, several initial treatments can be designed with the use of the biological scaffolds of the present disclosure, For example, joint-preservation surgery may include core decompression of the femoral head, sometimes in combination with autologous bone marrow cell transplantation, auto-bone grafting procedures with vascularized muscle pedicle or vascular anastomosis or non-vascularized autologous bone grafting procedures, and osteotomy.

Core decompression surgery can be used to relieve pain. In some examples, a 3-mm diameter drill may be used to produce multiple holes in the femoral head and filled with a biological scaffold of the present disclosure.

In some examples, autologous bone marrow cell transplantation may be employed. In these examples, the surgeon extracts more than 200 mL of iliac bone marrow blood, isolating mononuclear cells in vitro (without medium), and simply injecting them or implanting them with a biological scaffold of the present disclosure. Other exemplary examples can involve the use of autologous-bone grafting procedures involving the use of a biological scaffold of the present disclosure coating the vascularized muscle pedicle, vascular anastomosis or non-vascularized autologous bone grafts wherein the graft is coated at least on some of its surface with a biological scaffold. Vascularized bone grafts include deep iliac and superficial iliac vein grafts, the lateral femoral circumflex branch of the greater trochanter, the gluteal muscle branch of the greater trochanter bone and so on. Bone grafts with a muscle pedicle commonly utilize the femoral quadratus.

Allogeneic or autologous fibular grafts may be incorporated into a surgical procedure wherein the bone graft is coated or manufactured to contain a biological scaffold of the present disclosure.

Impaction bone grafting can utilize autologous bone or allograft bone, coated with a biological scaffold of the present disclosure with or without bone morphogenetic proteins and other bone osteoconductive and osteoinductive agents known in the art. In these bone grafting procedures an exemplary biological scaffold containing human platelet lysate and BM-DSCs. Osteotomy most commonly takes the form of femoral neck rotational osteotomy through the greater trochanter, varus subtrochanteric osteotomy and others.

In other examples, most patients with osteonecrosis of the femoral head will eventually have to undergo arthroplasty. With improvements in artificial joint design, materials and technique, the scope for j oint-preserving procedures is decreasing, whereas the indications for arthroplasty are expanding. The type of artificial joint can be selected according to the following recommendations: 1. Resurfacing has a limited role in subjects with ONFH because of the complications associated with metal-on-metal bearing surfaces. In each of these arthroplasty procedures, the artificial joint is coated with a biological scaffold of the present disclosure, for example, an exemplary biological scaffold containing human platelet lysate and BM-DSCs.

Also described herein is an illustrative biological scaffold which provides a cellular construct containing adipose stromal cells and/or adipose stem cells. In various related embodiments, the ASC containing biological scaffold comprises mammalian platelet lysate for example, a human platelet lysate made in accordance with the methods described herein, in the form of a liquid product mixed directly with ASCs derived from adipose tissue as a combination product that can be used as an injectable material or processed as a solid or semi-solid scaffold for tissue repair.

Also described herein is an illustrative biological scaffold which provides a cellular construct containing with BM-MSCs derived from bone marrow aspirates as a combination product that can be used as an injectable material or processed as a solid or semi-solid scaffold for tissue repair.

The present disclosure provides a kit or package which may be stored and shipped at - 20°C or -80°C which contains a frozen tube of the mammalian platelet lysate liquid product, one or more cryovials of SVF cells derived from adipose tissue, one or more bottles of medium of LaCell LLC products (LaCell LLC, New Orleans, Louisiana) specific for cell expansion (StromaQual), adipogenic differentiation (AdipoQual), chondrogenic differentiation (ChondroQual), or osteoblastic differentiation (OsteoQual), and tissue culture plates (6, 12, or 24 well) suitable for sterile cell growth and differentiation. The illustrative Kits described herein may be further modified by inclusion of proprietary adipogenic differentiation media designed to promote selectively white adipose tissue or brown/beige adipose tissue.

In various illustrative examples, the present disclosure provides a kit or package which may be stored and shipped at -20°C or -80°C which contains a frozen tube of the mammalian platelet lysate liquid product, one or more cryovials of ASCs derived from adipose tissue, one or more bottles of medium of LaCell LLC products specific for cell expansion (StromaQual), adipogenic differentiation (AdipoQual), chondrogenic differentiation (ChondroQual), or osteoblastic differentiation (OsteoQual), and tissue culture plates (6, 12, or 24 well) suitable for sterile cell growth and differentiation. The illustrative Kit may be further modified by inclusion of proprietary adipogenic differentiation media designed to promote selectively white adipose tissue or brown/beige adipose tissue.

In various illustrative examples, the present disclosure provides a kit or package which may be stored and shipped at -20°C or -80°C which contains a frozen tube of the mammalian platelet lysate liquid product, one or more cryovials of BM-MSC derived from bone marrow aspirates, one or more bottles of medium of LaCell LLC products specific for cell expansion (StromaQual), adipogenic differentiation (AdipoQual), chondrogenic differentiation (ChondroQual), or osteoblastic differentiation (OsteoQual), and tissue culture plates (6, 12, or 24 well) suitable for sterile cell growth and differentiation. The kit of the present disclosure may be further modified by inclusion of proprietary adipogenic differentiation media designed to promote selectively white adipose lineage or brown/beige adipose lineage differentiation.

In various illustrative examples, the present disclosure provides a kit or package which may be stored and shipped at -20°C or -80°C which contains a frozen tube of the mammalian platelet lysate liquid product, and a package of demineralized bone powder or hydroxyapatite/tricalcium phosphate or equivalent osteoinductive/osteoconductive bone powder alone or additionally containing one or more cryovials of SVF Cells, and/or one or more cryovials of ASCs, and/or one or more cryovials of BM-MSCs. The contents of the kit are combined by the orthopedic, plastic, or craniofacial surgeon at the point of care or operating room and delivered to the patient's surgical or injury site as a paste or solidified combination product to promote bone regeneration.

In some examples, kits are provided which may be stored and shipped at -20°C or - 80°C which contains a frozen tube of the mammalian platelet lysate in the form of a liquid product and nozzle device without or with cryovials of the SVF cells, and/or ASC, and/or BM-MSC. The resulting combination product comprising the liquid biological scaffold is designed for spray delivery to the skin or wound site of a patient following chemical, electrical, radiation, or thermal burn or injury. The product of the kit can be delivered as the mammalian platelet lysate alone, or in combination with SVF cells, and/or ASCs, and/or BM-MSCs.

In some embodiments products containing a biological scaffold of the present disclosure can include a package which contains a sterile bandage, synthetic mesh or gauze material and a biological scaffold of the present disclosure. In an illustrative example, the bandage, mesh or gauze will already have been immersed in and coated with the mammalian platelet lysate during the manufacturing process and subsequently stored as a frozen combination product or as a lyophilized product that can be stored for extended periods at room temperature or 4°C. In use, the bandage, mesh or gauze can then be coated with SVF cells subsequently applied to the tissue requiring coverage of the bandage, mesh, or gauze material. For example, in cartilage or muscle tissue applications meshes can be impregnated with the mammalian platelet lysate and during surgery the tissue to be affixed with the mesh may be added to a specific cell fraction that is operable to be induced into the tissue the mesh is in contact, for example, in hernia reparations and other abdominal and urogenital applications in which a mesh may be affixed to muscle or cartilage materials. Alternatively, the bandage or gauze material and the liquid mammalian platelet lysate may be shipped separately in the package, allowing the physician or surgeon to combine them together with the SVF cells directly at the point of care. The bandage will then be administered directly to the wound site or injury of the patient.

In some examples, the present disclosure provides a package which contains a cryopreserved container of mammalian platelet lysate and one or more cryovials of ATDCF cells, for example, SVF Cells, and/or ASCs, and/or BM-MSCs. These materials are shipped to the user on dry ice and stored at -20°C or -80°C prior to use. The materials are designed to be used with a 3D printer to allow for the manufacture of a customized three-dimensional cell/scaffold structure. In some embodiments, different regions, sections and/or surfaces may be imprinted with the same biological scaffold material or different biological scaffold materials.

## C. Exemplary Uses Of Biological Scaffolds

In some examples, mammalian platelet lysate can be combined with human adipose tissue-derived stromal vascular fraction (SVF) cells, adipose-derived stromal/stem cells (ASC), and/or bone marrow-derived mesenchymal stromal/stem cells (BM-MSC) to create a combination product suitable for the study of adipose tissue or bone and bone marrow biology. The combination product could be cultured in vitro in the presence or absence of cocktail agents known to promote adipogenesis (AdipoQual), chondrogenesis (ChondroQual), or osteogenesis (OsteoQual). Combining the mammalian platelet lysate with SVF cells, ASC, or BM-MSC in the presence of AdipoQual leads to the formation of three-dimensional adipose tissue structures and adipose tissue function that can be evaluated on the basis of gene, protein, and small molecule expression, as well as glucose uptake, lipolysis, metabolic activity, and other adipose tissue functional assays. Combining mammalian platelet lysate with SVF cells, ASC, or BM-MSC in the presence of ChondroQual leads to the formation of three-dimensional cartilage tissue structures and function that can be evaluated on the basis of gene, protein, and small molecule expression, as well as glycosaminoglycan production, metabolic activity, and other cartilage tissue functional assays. Combining mammalian platelet lysate with SVF cells, ASC, or BM-MSC in the presence of OsteoQual leads to the formation of three-dimensional bone and bone marrow tissue structures and function that can be evaluated on the basis of gene, protein, and small molecule expression, as well as extracellular matrix mineralization, calcium uptake, metabolic activity, and other bone tissue functional assays such as lymphohematopoiesis.

In some examples, the biological scaffolds of the present disclosure can be used as a combination kit product for the development, extended culture, and expansion of human adipose tissue derived cells for the purpose of research within normal or diseased adipose tissue. The biological scaffold can be initiated and cultured at 37°C to create an adipose cellularized three-dimensional scaffold. In various examples, the present disclosure provides kits which may contain1 components required for independent setup, maintenance, and assay endpoints to evaluate adipose tissue gene, protein, and small molecule expression, and tissue functionality. In some examples, the kit of the present disclosure can include a shipping package that contains vial(s) of cryopreserved primary ATDCF cells, mammalian platelet lysate, culturing medium, a substrate, and instructions. In some examples, white or brown/beige adipose differentiated tissue bioscaffolds can be customized to include SVF cells, and/or ASCs, and/or BM-MSC as well as medium specifically designed to promote the formation and differentiation of white or brown/beige adipose tissue depots.

The white and/or brown/beige adipose differentiated tissue bioscaffolds can be customized with SVF cells, and/or ASCs and/or BM-MSCs as a combination kit product or offered as a contract service for purpose of drug discovery research within normal or diseased adipose tissue. The cells employed can be harvested from donors in good health or those with specific diseases or conditions of interest, such as obesity or Type 2 Diabetes Mellitus (T2DM). The white and/or brown/beige adipose differentiated tissue bioscaffolds combination product/service can be cultured at 37°C in the presence or absence of pharmaceutical or cosmeceutical agents, and the early and late tissue response can be measured. Assay endpoints can be measured to evaluate adipose tissue gene, protein, and small molecule expression, cell biological, chemical, toxicological, and functionality response to the agents. In some exemplary examples, white and/or brown/beige adipose differentiated tissue bioscaffolds may include a shipping package that contains vial(s) of cryopreserved primary cells, mammalian platelet lysate, culturing medium, a substrate, and instructions. The service can consist of adipose tissue culture setup, maintenance, and the addition of compounds for the treatment of disease conditions. The service can also include the evaluation of adipose tissue response to the chemical compounds by assay.

: Biological Scaffold product for creation of a humanized murine fat depot model.

In some embodiments, the biological scaffold can be used to create murine models of human fat or adipose depots. Biological scaffolds (containing human SVF cells and/or containing human ASCs) can be injected subcutaneously in volumes of 250 µl or larger under the epidermis/dermis of either immunocompetent or immunodeficient mice using a sterile syringe and 19 gauge needle. The subcutaneous injection sites may be sealed with Vetbond or an equivalent material to prevent subsequent leakage. The implants would be allowed to mature for a period of up to 12 weeks. During this period, the implants will acquire the characteristics of a differentiated adipose depot. The human derived cells will constitute a percentage of the cells within these adipose depots for up to 12 weeks or longer. The mice can be fed a diet composed of 45% or higher fat content (high fat diet) to enhance the growth and differentiation of the humanized adipose depot. Likewise, the mice can be treated with small molecule chemicals or peptides/proteins to evaluate their effect on the metabolism of human adipose tissue under physiological conditions. The humanized fat depots may be monitored using standard scientific approaches including, but not limited to, cell biology, histology, in vivo imaging, metabolic, and related methods.

Biological scaffolds of the present disclosure may be utilized for the creation of patient derived xenografts or tumors in vivo or in vitro.

Biological scaffolds containing human adipose tissue derived SVF cells or ASCs can be used to promote the culture and expansion of human tumors. The biological scaffold of the present disclosure can be combined with a tumor cell line or tumor fragment (1 to 10 mm$^3$ volume) and cultured in vitro at 37°C to create an adipose cellularized three dimensional tumor containing scaffold. This combination biological scaffold product could then provide a stromal/vascular environment to promote the long term viability, proliferation, and expansion of human derived primary or metastatic tumors, obtained either before or after patient exposure to chemotherapy or radiotherapy or immunotherapy. Tumor fragments of 1 to 10 mm$^3$ would be implanted directly on the combination adipose cell biological scaffold in vitro or injected with the biological scaffold in liquid form in vivo under the skin of an immunodeficient mouse. These tumors, either in vitro or in vivo, would be maintained for periods of 1 to 12 months and monitored for growth based on volume, size, cell number, and secretion of factors. The tumors could be derived from breast cancer, prostate cancer, bone cancer, colon cancer, or other sources of malignancy.

Biological scaffolds of the present disclosure may be utilized for the development of a human bone or human bone marrow in vitro or in vivo in a murine model.

The biological scaffold containing BM-MSCs, described herein, with and without demineralized bone powder or hydroxyapatite/tricalcium phosphate or equivalent osteoinductive/osteoconductive bone powder alone can be used to create an in vitro or in vivo construct suitable for the study of human hematopoiesis and osteogenesis. The biological scaffold could be cultured in vitro in the presence of cytokines and growth factors known to promote lymphohematopoiesis and monitored for the detection and expansion of non -adherent and adherent cells expressing surface antigens associated with the lymphoid and myeloid lineages. Alternatively, the combination product could be implanted into the cleared marrow cavity or subcutaneously in an immunodeficient mouse and subsequently monitored for the production of human hematopoietic and osteogenic cells based on flow cytometry and immunohistochemical or molecular biological analysis of the hard tissue. The resulting in vitro and in vivo models could be used to design experiments of human blood and bone formation.

Biological scaffolds containing any one or more ATDCF cells, for example, SVF Cells, and/or ASCs, and/or BM-MSCs may be utilized for the development of a bone in a clinical setting. In some examples, biological scaffolds containing any one or more ATDCF cells, for example, SVF Cells, and/or ASCs, and/or BM-MSCs can be used by orthopaedic, plastic or craniofacial surgeons to repair traumatic, inborn, or surgical bone defects in weight bearing and non-weight bearing sites. The combination of a biological scaffold containing an ATDCF (SVF cells, and/or ASCs and/or BM-MSCs), and osteoinductive/osteoconductive ceramic materials may be combined at the point of care to create a paste that is delivered to the bone defect site. The combined material may be implanted into and confined within the dimensions of the defect by the surgeon using the surrounding tissue or a suitable conduit or membrane. The surgeon would stabilize the defect using open reduction/internal fixation approaches appropriate for standard of care. The combination biological scaffold described above, would then promote and accelerate bone repair, recovery, and remodeling during the subsequent postoperative period as monitored non-invasively using radiological imaging modalities.

In some examples, biological scaffolds of the present disclosure may be formulated for spraying for the treatment of burns secondary to chemical, electrical, flame, radiation, or thermal injury.

In some examples, a biological scaffold formulated for administration as a spray can be used by general, plastic and reconstructive, and trauma surgeons to treat first-degree, second-degree, or third-degree burns of the skin and underlying tissues in the acute and chronic settings. The spray formulation containing the biological scaffold may comprise a nozzle operable to provide controlled delivery of the biological scaffold alone as a spray of small droplets/particles to the injury site from a distance of 10 cm or less above the surface. The surgeon or other health professional providing the service can monitor the degree of wound surface coverage based on visual inspection. Where appropriate, the spray formulation of the biological scaffold and the device which applies said biological scaffold can deliver the autologous or allogeneic cells, which can be but are not limited to SVF cells and ASC derived from adipose tissue, BM-MSC derived from bone marrow, or optionally in addition, dermal fibroblasts, epidermal cells, and/or keratinocytes derived from skin. The application of sprayed biological scaffold with or without cell supplementation would be designed to accelerate and enhance the formation of an intact new skin containing pigment epithelial cells, hair follicles, sebaceous glands, sweat glands, immune cells and appropriate epidermal/dermal and underlying tissue architecture necessary to preserve all of the functions attributed to a health integument, including but not limited to maintenance of body fluid evaporation and conservation, immune barrier, vitamin D metabolism, and UV radiation protection.

Various formulations, products and applicators containing a biological scaffold of the present disclosure may be used for the treatment of pressure ulcers or injuries or diabetic ulcers.

In various examples, biological scaffolds formulated as gels, solutions and applied to semi-solid or solid substrates such as bandages, gauze and meshes can be used individually or in combination by general and plastic and reconstructive surgeons to treat acute and chronic skin wounds involving the epidermis, dermis, and/or underlying tissues (adipose, skeletal muscle, bone) such as pressure injuries or pressure ulcers secondary to ischemia/reperfusion injuries in elderly and paraplegic/quadriplegic patients or diabetic ulcers secondary to ischemia resulting from compromised vascularization of distal extremities in patients with either Type 1 or Type 2 Diabetes Mellitus. Prior to application of the biological scaffold, the wound area may be debrided and the surrounding skin surface washed with 70% ethanol and betadine or an equivalent anti-bacterial cleanser. The mammalian platelet lysate or biological scaffold containing products may be injected under aseptic technique directly into the base of the chronic skin wound/pressure ulcer/diabetic ulcer using a #19 or #21-gauge needle in a fan-like manner covering the entire wound surface. Injections would be performed by entering the skin from outside the perimeter of the wound site itself to avoid any risk of contamination. The mammalian platelet lysate or biological scaffold containing injections can be repeated at biweekly, monthly or less frequent intervals based on the clinical judgment of the surgeon. Additionally, the mammalian platelet lysate or biological scaffold containing products, for example, the mammalian platelet lysate, biological scaffold or products containing the biological scaffold, for example, a bandage, a mesh or a dressing, can be delivered or placed topically to the surface of the debrided pressure ulcer or diabetic ulcer injury site. This would then be covered with an aseptic Adaptic or Tegaderm bandage to seal the gelled product or bandage from the environment. The topical therapy can be repeated up to three times weekly based on the judgment of the surgeon. Additionally, the topical therapy can be delivered in combination with negative pressure wound vacuum according to current standard of care practices. The application of mammalian platelet lysate, biological scaffold or products containing the biological scaffold, for example, a bandage, a mesh or a dressing will accelerate and enhance the rate of chronic skin wound/ulcer closure and healing based on direct observation, histological architecture, and functional outcomes measures such as skin integrity, water loss/evaporation, immune barrier, and physical appearance.

Also described herein are methods for preparing a biological scaffold of the invention. For example, a mammalian (for example, a human) platelet lysate usage and one or more ATDCF cells are admixed to form a biological scaffold. In some examples, a biological scaffold containing any one or more ATDCF cells, for example, SVF Cells, and/or ASCs, and/or BM-MSCs cell types selected from Stromal Vascular Fraction (SVF) Cells, and/or Adipose-derived Stromal/Stem Cells (ASC) and/or Bone Marrow-derived Mesenchymal Stromal/Stem Cells (BM-MSC) are mixed together to form the biological scaffold. In an exemplary procedure for the preparation of such biological scaffolds, the following steps may be employed: Perform all steps within a biological safety cabinet unless otherwise indicated. Obtain desired amount concentrated expired human platelets in 15mL or 50mL conical tube. If not using immediately for 3D cell culture, store at -20°C before making the human platelet lysate preparation by three freeze/thawing cycles. Perform 3 freeze/thaw cycles to lyse platelets by osmosis. This will release maximum yield of growth factors for cell cultures. Centrifuge 8,000 x g for 20 mins at room temperature. Remove the solid (white) top layer of human platelet membranes by aspiration. The remaining liquid infranatant comprises the human platelet lysate product. Prepare human platelet lysate supplemented media by adding 7.5% (v/v) human platelet lysate to 1% antibiotic/antimycotic and 91.5% Dulbecco's Modified Essential Medium or other related cell culture medium containing necessary nutrients, amino acids, and growth factors for cell culture. Note; The percentage concentration of human platelet lysate can be varied by altering the corresponding content of DMEM or other related cell culture medium appropriately while continuing to supplement with 1% antibiotic/antimycotic. After mixing, do not warm in water bath. Maintain at room temperature. Activate gelation (formation of a thick matrix) conducive to cell secretion of extracellular matrix, proliferation, and differentiation by combining prepared human platelet lysate with cell population or mixture of cell populations. After combining cells and human platelet lysate, add plate to incubator for 20 mins for activation of gelation. Proceed to proliferation, differentiation, implantation into mice, or other measured endpoints.

**Biological Scaffolds For Use In Tissue Reconstruction.**

In some examples, the biological scaffolds of the present disclosure may be used for replacement, augmentation and enhancement of tissue reconstruction, and for cosmetic applications. In an exemplary method, surgeons or physicians may elect to treat patients afflicted by scars or defects secondary to trauma, surgery, or tumor resection as well as inborn errors such as Poland's syndrome or Treacher Collin syndrome using products incorporating the biological scaffolds described herein. In one such example, a surgeon may use a cryopreserved container (5 or 10 mL) stored at -20° C containing the mammalian platelet lysate manufactured under current Good Manufacturing Practice (cGMP) and approved for clinical use. In some examples, the platelet lysate is a human platelet lysate. However, platelets from other mammalian species could be utilized and the human platelets can be autologous or allogeneic. In some examples, the a mammalian (for example, a human) platelet lysate can be used alone or in combination with autologous, patient derived stromal vascular fraction (SVF) cells isolated from lipoaspirate tissue collected at the time of therapy using a closed system apparatus or device such as those manufactured by Tissue Genesis (Honolulu HA) or Cytori Therapeutics (San Diego CA). Alternatively, the human platelet lysate can be substituted with human platelet lysate in the form of a liquid product mixed directly with ASCs derived from adipose tissue as a combination product that can be used as an injectable material or processed as a solid or semi-solid scaffold for tissue repair.

In some related examples, the biological scaffold for use in tissue reconstruction or augmentation can include a human platelet lysate in the form of a liquid product mixed directly with BM-MSCs derived from bone marrow aspirates as a combination product that can be used as an injectable material or processed as a solid or semi-solid scaffold for tissue repair to create an allogeneic combination cellular therapeutic using allogeneic adherent adipose or bone marrow derived cells culture expanded under current Good Manufacturing Practice (cGMP) protocols. The combination of liquid human platelet lysate with or without supplementing autologous or allogeneic cells can then be used as an injectable material by the surgeon or physician. The practitioner would aspirate the liquid human platelet lysate with or without autologous or allogeneic cells (SVF cells, and/or ASCs and/or BM-MSCs) into a sterile syringe. The liquid material would then be injected subcutaneously into the subdermal layer below the tissue to be treated, for example, a wound, a scar or skin or tissue defect. The liquid may be delivered using a #19 or #21 gauge needle in a fan-like pattern from the injection site to thoroughly infiltrate the underlying dermis. The practitioner would inject sufficient volume to achieve the desired cosmetic effect with respect to the contours of the wound or defect site. The procedure could be performed singly or on multiple repeat occasions to achieve the desired cosmetic and reconstructive outcome.

In one illustrative example, the mammalian platelet lysate and/or the biological scaffold may be used for tissue reconstruction and cosmetic enhancements. An illustrative method may include: The mammalian platelet lysate is provided to the surgeon, operating room, or other health delivery center as a frozen sterile aliquot containing a volume of 1 to 20 ml of mammalian platelet lysate packaged in accordance with FDA and cGMP guidelines. The clear container will be suitable to withstand the freeze/thawing cycle without risk of fracture. The mammalian platelet lysate container will be thawed in a 37o C bath or dry oven rapidly until the ice crystals have nearly all disappeared. The mammalian platelet lysate container will be opened within the surgical theater and the liquid contents aspirated into a sterile 1 to 20 ml syringe. If applicable, the surgeon will likewise thaw and aspirate a cryopreserved vial of allogeneic SVF cells, ASC, or BM-MSC. Alternatively, the surgeon will harvest the patient's own lipoaspirate as a source of autologous SVF cells. Either the autologous or allogeneic cells will then be mixed directly with the mammalian platelet lysate liquid by connecting two syringes via a sterile stop-cock to create a combination biological scaffold product. Using a #19 gauge or smaller needle, the surgeon will then inject the mammalian platelet lysate or biological scaffold product subcutaneously into the patient using a modification of the Coleman technique whereby small volumes of liquid are injected upon withdrawal of the needle and delivery throughout the subcutaneous tissue in a fan-like manner. The surgeon can deliver the mammalian platelet lysate or biological scaffold product by injection to the subcutaneous and supramuscular breast tissue to achieve volume enhancement and breast augmentation for cosmetic applications; to the base of scars or disfigurements on the face or elsewhere to remove volume deficits and improve appearance or; to the base of debrided pressure ulcers or diabetic ulcers to enhance wound closure and healing.

## Applications of the biological scaffolds for drug discovery.

In some examples, the biological scaffolds described herein may be utilized for the preparation of cellular constructs useful in drug discovery. In one example, the cellular construct comprising the biological scaffold may contain a container of mammalian platelet lysate either in liquid or semi-solid (gelatinous) or in solid form (the scaffold applied to a solid substrate), a substrate for culturing white or brown adipose, and reagent A. Illustrative methods for using such cellular constructs for drug discovery may include the steps: Prepare the 3D tissue culture using the following instructions: Prepare a solution of mammalian platelet lysate supplemented media by adding 25% (v/v) of the mammalian platelet lysate to 1% antibiotic/antimycotic and 74% Dulbecco's Modified Essential Medium or other related cell culture medium containing necessary nutrients, amino acids, and growth factors for cell culture. After mixing, do not warm in water bath. Maintain at room temperature. Thaw stromal vascular fraction (SVF), bone marrow-derived mesenchymal stem/stromal (BM-MSC), or adipose stromal/stem (ASC) cells provided in cryovials from liquid nitrogen in 37°C water bath until the moment ice disappears. Rinse each cryovial once with 1mL standard Stromal Medium. Centrifuge tube at 300x g (1200 rpm) for 5 minutes. Re-suspended the cell pellet in hPL supplemented Medium at a concentration of $5x10^5$ cells per mL. Immediately plate the cell/hPL mixture. Mixture will immediately gel when placed in $CO_2$ incubator. Maintain 3-D culture by replacing evaporated culture media every 7 days. Remove the old media in the cultures by aspiration via a 1mL micropipette. Replace with fresh media by adding a half-volume amount of the hPL-media to the top corner of the gel. Add desirable pharmaceutical agents directly to cultures in desired concentrations to perform the drug discovery assay. Test endpoints of interest, including imaging of cell population changes, protein expression and secretion, gene expression, glucose uptake, lipolysis, or metabolic activity.

## D. EXAMPLES

## Reference example 1. Preparation Of Components Of A Biological Scaffold

Isolation of Bone Marrow Mesenchymal Stem Cells (BM-MSC)

To isolate BM-MSC, first obtain bone marrow aspirate that has been anti-coagulated with heparin or citrate to prevent clotting. Maintain the bone marrow aspirate on wet ice at 4o C prior to use and perform all steps with open containers within a BSL2 biological safety cabinet. Thoroughly mix Ficoll Paque solution (GE Healthcare™ Ficoll-Paque™ PREMIUM, 1.078g/mL). Aliquot 20 ml of Ficoll Paque solution to a sterile 50 ml conical tube. Gently layer 10 ml of bone marrow aspirate onto the top of the Ficoll Paque layer and avoid any mixing. After sealing the tube, centrifuge for 30 minutes at 500 X g at room temperature without any braking using a benchtop Sorvall™ Legend™ centrifuge. After returning the conical tube to the BSL2 biological safety cabinet, aspirate off the upper clear plasma layer and aliquot to a fresh sterile 50 ml conical tube. Next, aspirate the buffy coat layer of mononuclear cells from above the Ficoll Paque layer. Transfer the mononuclear cells to a sterile 15 ml conical tube and suspend in 3 volumes of sterile Phosphate Buffered Saline (PBS) solution. Centrifuge the tube for 10 minutes at 200 X g at room temperature. Aspirate the supernatant from the pelleted mononuclear cells after washing away any contaminants and Ficoll-Paque. Resuspend the pelleted mononuclear cells in a volume of 1 to 10 ml of LaCell Stromal Medium™. Remove a 10 $\mu$l and mix with an equal volume of LaCell's Live Dead Assay Solution™ containing acridine orange/ethidium bromide and count the live (green) and dead (red) nucleated cells on a fluorescent microscope (Motic AE30 Epifluorescent Microscope). Based on the percentage of live cells and the total cell count per milliliter, calculate the total number of nucleated cells using the formula:

$$\text{Total Live Cells} = \text{Percentage Live Cells X Volume (in ml) X Total Cell Count per ml}.$$

Suspend the mononuclear cells at a concentration of between $10^5$ to $10^6$ per ml of LaCell Stromal Medium™. Seed the cell suspension at a density of $5 \times 10^3$ to $5 \times 10^4$ per square centimeter in T25, T75, or T175 flasks with volumes of 15, 25, or 35 to 50 ml of LaCell Stromal Medium™, respectively. Culture the flasks in a 37°C humidified 5% $CO_2$ incubator for a period of 1 to 3 weeks. Replace 50% of the medium every 2 to 3 days with fresh LaCell Stromal Medium™ or with 1:1 fresh: BM-MSC conditioned LaCell Stromal Medium™. When the BM-MSC reach 70 to 90% confluence, passage the cells by digestion with 0.25% trypsin at 37°C in a humidified 5% $CO_2$ incubator. Monitor the adherent cells in the flask by phase contrast microscopy to insure that trypsin digestion has released a single cell suspension. After 5 to 20 minutes of trypsin digestion, add an equal volume of LaCell Stromal Medium™ to quench the enzyme digestion reaction and transfer the total cell volume to a sterile 15 ml or 50 ml conical tube. Remove a 10 $\mu$l volume of the cell suspension and mix with an equal volume of trypan blue solution. Determine the total cell number and the percentage of live cells by counting the clear (live) and blue (dead) cells using a Neubauer hemocytometer under phase contrast microscopy (Motic). Centrifuge the cell suspension at 300 X g at room temperature. Aspirate the supernatant and resuspend in either (a) fresh LaCell Stromal Medium™ at a concentration of $15 \times 10^3$ to $150 \times 10^3$ live cells per ml for continued passage and culture at a density of 1 ml of medium per 5 square centimeters of flask surface area or (b) LaCell Cryopreservation Medium™ at a concentration of $10^6$ to $10^7$ live cells per ml for immediate cryopreservation aliquoted to 1.8 ml cryopreservation tubes using a Mr. Frosty™ alcohol bath freezer container placed overnight at -80°C prior to transfer of the cryopreservation **tubes** to a liquid nitrogen dewar storage container. The quality and characteristics of the BM-MSC can be monitored in process using the following assays:

Colony Forming Unit Fibroblast (CFU-F) assay for analysis of adherent cell numbers and proliferative potential.

Adipogenic, Chondrogenic, and Osteogenic differentiation assays as induced by culture in the presence of LaCell's AdipoQual™, ChondroQual™, or OsteoQual™ Differentiation Induction Medium for periods of 14 days and detected by staining for Oil Red O (Adipoctyes), Alcian Blue (Chondrocytes) or Alizarin Red (Osteoblasts).

Flow Cytometry with detection of a panel of surface antigens including but not limited to: Stromal Markers CD29, CD44, CD73, CD90, CD105; Hematopoietic Markers CD11b, CD14, CD45; Stem Cell Markers CD34; Pericytic Markers CD146; Endothelial Marker CD31.

## Example 2. Isolation of Stromal Vascular Fraction (SVF) Cells and Adipose-derived Stromal/Stem Cells (ASC)

To isolate SVF cells, first obtain a volume of at least 100 ml of lipoaspirate or 50 grams of subcutaneous adipose tissue from patients undergoing elective liposuction or abdominoplasty. The tissue can be stored at room temperature for up to 24 hours after the surgical procedure to allow for shipping or transit to the laboratory. Transfer the tissue upon receipt to the laboratory into a sterile BSL2 biological safety cabinet. If the tissue is received intact (after abdominoplasty), mince the tissue thoroughly into fragments of 2 to 3 mm3 using either two scalpels or two pairs of fine scissors that have been sterilized or autoclaved prior to use. After mincing the tissue or aliquoting the lipoaspirate, transfer the 100 ml volume of tissue to a sterile 250 ml capped Nalgene™ plastic centrifuge tube. Add an equal volume of sterile PBS and allow the tissue and liquid phases to separate over a 2 to 5 minute period. Using a sterile 1 ml aspiration pipet, remove the bloody layer of PBS from beneath the floating tissue layer. Repeat this procedure 3 to 5 times until the PBS infranatant is no longer bloody but at most, a straw color.

Prepare a solution of 0.1% Collagenase Type I (Worthington Biochemicals Cat #CLS1, Lakewood NJ), 1% bovine serum albumin (BSA) fraction V (Fisher Scientific) in PBS by weighing out 100 mg of Collagenase Type 1 and 1 gram of BSA and dissolving in 100 ml of PBS and sterile filtering through a 0.22 micron filter unit within the BSL2 biological safety cabinet. This solution is then warmed to 37°C in a heated water bath or incubator. Mix the 100 ml of the 0.1% Collagenase/1% BSA solution in PBS with the 100 ml volume of adipose tissue. Seal the 250 ml centrifuge bottle and place inside a Brunswick shaker incubator at 37o C and rotating at 200 to 220 rpm for 50 to 70 minutes. After completing the incubation and rocking, the adipose tissue fragments should display signs of digestion and loss of architectural integrity. Centrifuge the contents of the digest at 300 X g at room temperature for 5 minutes. Resuspend the resulting pellet and repeat the centrifugation step. Return the centrifuge tube to the BSL2 biological safety cabinet and aspirate the floating adipose tissue and supernatant. Resuspend the pelleted SVF cells in a volume of 15 mL of LaCell Stromal Medium™. Remove an aliquot of 10 $\mu$L of cell suspension and mix with an equal volume of LaCell's Live Dead Assay Solution™ containing acridine orange/ethidium bromide and count the live (green) and dead (red) nucleated cells on a fluorescent microscope (Motic AE30 Epifluorescent Microscope). Based on the percentage of live cells and the total cell count per milliliter, calculate the total number of nucleated cells using the formula: Total Live Cells = Percentage Live Cells X Volume (in ml) X Total Cell Count per ml.

At this point, the SVF cells optionally can be: (a) cryopreserved by pelleting the total cell volume by centrifugation at 300 x g at room temperature for 5 minutes and resuspension in LaCell Cryopreservation Medium™ at a cell concentration of $10^6$ to $10^7$ per ml, aliquoting in 1 ml volumes to 1.8 ml cryopreservation vials, and freezing in a Mr. Frosty™ container overnight at -80°C prior to transfer to liquid nitrogen dewar for long term storage or; (b) culture expansion to obtain adherent adipose-derived stromal/stem cells (ASC) by resuspending the SVF cell volume isolated from 100 ml of lipoaspirate in a total volume of 105 ml of LaCell Stromal Medium™ and aliquot 35 ml to each of three T175 flasks. Incubate the flasks in a 37°C in a humidified 5% $CO_2$ incubator for 24 to 72 hours. Determine the extent of cell adherence by inspection under phase contrast microscopy using a Motic microscope at 20 X magnification. Within a BSL2 biological safety cabinet, aspirate the medium from the flask before twice washing the adherent cells with a 20 ml volume of sterile PBS pre-warmed to 37°C. Re-feed the adherent cell population with 35 ml of fresh LaCell Stromal Medium™ or with 35 ml of 1: 1 fresh: ASC conditioned LaCell Stromal Medium™ per T175 flask. Maintain the flasks in culture while feeding with fresh medium or 1: 1 fresh: ASC conditioned medium every 2-3 days until reaching 70% to 90% confluence. At this point, it is necessary to passage the ASCs by digestion with 0.25% trypsin at 37°C in a humidified 5% $CO_2$ incubator. Monitor the adherent cells in the flask by phase contrast microscopy to insure that trypsin digestion has released a single cell suspension. After 5 to 20 minutes of trypsin digestion, add an equal volume of LaCell Stromal Medium™ to quench the enzyme digestion reaction and transfer the total cell volume to a sterile 15 ml or 50 ml conical tube. Remove a 10 $\mu$l volume of the cell suspension and mix with an equal volume of trypan blue solution. Determine the total cell number and the percentage of live cells by counting the clear (live) and blue (dead) cells using a Neubauer hemocytometer under phase contrast microscopy (Motic). Centrifuge the cell suspension at 300 X g at room temperature. Aspirate the supernatant and resuspend in either (a) fresh LaCell Stromal Medium™ at a concentration of 15 X $10^3$ to 150 X $10^3$ live cells per ml for continued passage and culture at a density of 1 ml of medium per 5 square centimeters of flask surface area or (b) LaCell Cryopreservation Medium™ at a concentration of $10^6$ to $10^7$ live cells per ml for immediate cryopreservation aliquoted to 1.8 ml cryopreservation tubes using a Mr. Frosty™ alcohol bath freezer container placed overnight at -80°C prior to transfer of the cryopreservation tubes to a liquid nitrogen dewar storage container. The quality and characteristics of the ASC can be monitored in process using the following assays:

Colony Forming Unit Fibroblast (CFU-F) assay for analysis of adherent cell numbers and proliferative potential.

Adipogenic, Chondrogenic, and Osteogenic differentiation assays as induced by culture in the presence of LaCell's AdipoQual™, ChondroQual™, or OsteoQual™ Differentiation Induction Medium for periods of 14 days and detected by staining for Oil Red O (Adipoctyes), Alcian Blue (Chondrocytes) or Alizarin Red (Osteoblasts).

Flow Cytometry with detection of a panel of surface antigens including but not limited to: Stromal Markers CD29, CD44, CD73, CD90, CD105; Hematopoietic Markers CD11b, CD14, CD45; Stem Cell Markers CD34; Pericytic Markers CD146; Endothelial Marker CD31.


## Reference Example 3. Preparation Of Exemplary Biological Scaffolds

In another experimental example, an adipose-tissue device comprising SVF and a silk bioscaffold is described. Progenitors derived from the stromal vascular fraction (SVF) of white adipose tissue (WAT) possess the ability to form clonal populations and differentiate along multiple lineage pathways. However, the literature continues to vacillate between defining adipocyte progenitors as "stromal" or "stem" cells. Recent studies have demonstrated that a non-pericytic subpopulation of adipose stromal cells, which possess the phenotype, CD45-/CD31-/CD146-/CD34+, are mesenchymal, and suggest this is an endogenous progenitor subpopulation within adipose tissue. Frazier, et al. demonstrated that stromal vascular fraction (SVF) cells and culture expanded adipose stromal/stem cells (ASC) ubiquitously expressing the GFP transgene (GFP-Tg) could be fractionated by flow cytometry. Both freshly isolated SVF and culture expanded ASC were seeded in 3-D silk scaffolds, implanted subcutaneously in wild-type hosts, and serially transplanted successfully over one initial, and two serial implants (2° implants). Six-week WAT constructs were removed and evaluated for the presence of GFP-Tg adipocytes and stem cells. Flow cytometry, quantitative polymerase chain reaction, and confocal microscopy demonstrated GFP-Tg cell persistence, proliferation, and expansion, respectively. Glycerol secretion and glucose uptake assays revealed GFP-Tg adipose was metabolically functional. Constructs seeded with GFP-Tg SVF cells or GFP-Tg ASC exhibited higher SVF yields from digested tissue, and higher construct weights, compared to non-seeded controls. Constructs derived from CD146- CD34+-enriched GFP-Tg ASC populations exhibited higher hemoglobin saturation, and higher frequency of GFP-Tg cells than unsorted or CD29+ GFP-Tg ASC counterparts. These data demonstrated successful serial transplantation of non-pericytic adipose derived progenitors that can reconstitute adipose tissue as a solid organ.

In one embodiment the present disclosure provides an adipose-tissue device comprising SVF and a decellularized extracelleular adipose matrix (ECM). Three methods were compared for optimal adipose tissue decellularization. Method one (M1) is an enzyme based method developed by the laboratory of Dr. Lauren Flynn, Ontario, Canada. Method two (M2) is a novel, solvent-free method that is an improvement of a solvent-based method reported by Ji Suk Choi, Hanyang University, Republic of Korea and Kinam Park, Purdue University. Method three (M3) is based on the preparation of the commercially available, tumor-derived extracellular matrix product, Matrigel™.

Human adipose tissue was obtained from consenting abdominoplasty patients under an IRB approved protocol. These studies compared alternative decellularization processes with respect to the desired properties of a final ECM product. Bioscaffolds were evaluated for DNA depletion, ECM composition, physical structure, and protein content. Decellularization was considered confirmed by a lack of cell nuclei present upon H&E staining, and further supported by the absence of residual DNA- based on spectrophotometric analysis. Extensive collagen composition was observed via trichrome staining. Scanning electron microscopy (SEM) revealed a complex fibrous physical constitution. Mass spectroscopy proteomic analyses identified 25 proteins as definitively present (having 2+ peptide sequence hits) in the M1 scaffolds, 143 in M2, and 102 in the M3 scaffolds, while the native tissue presented 155. Some of the structural proteins displayed relative enrichment as a result of decellularization; these included collagen type VI, fibrillin, and laminin. The results herein extend the identification of the protein components of adipose tissue-derived bioscaffolds and further define the functional microenvironment that promotes ASC adhesion, proliferation, and differentiation within a solvent-free method that is superior to the current technology.

**Reference example: experimental for Silk-fibroin-based biological scaffolds**

Materials and Methods

Mice

Animal studies were performed under the veterinary supervision of the Department of Comparative Medicine of the Tulane University School of Medicine under a protocol reviewed and approved by the Institutional Animal Care and Use Committee in accordance with federal, state, and National Institute of Health policies and regulations. SVF cells and ASC were isolated from 8 to 12 week male C57BL/6-Tg (UBC-GFP) 30cha/J mice (human ubiquitin C promoter driven green fluorescent protein (GFP) transgene, Jackson Laboratory, Bar Harbor, ME, https://www.jax.org) according to published methods. GFP transgene (GFP-Tg) ASC retain expression of the GFP transgene upon isolation and in vitro expansion out to at least 10 passages, and display a cell doubling time of between 2 and 2.5 days. For initial characterization, cells were examined for expression of markers CD11b (Mac-1$\alpha$; Integrin alpha M), CD29 ($\beta$1 integrin), CD34 (mucosialin), CD45 (leukocyte common antigen; Ly5), CD90 (Thy-1), and Sca-1 (stem cell antigen 1; Ly6A/E).

Adipose Tissue Harvest and SVF Cell Preparation

Subcutaneous inguinal white adipose tissue (iWAT) from 8 to 12 week male C57BL/6-Tg (UBC-GFP) 30cha/J mice was isolated, minced, and digested with collagenase for 60 minutes according to a published protocol from our laboratory. Briefly, the iWAT SVF pellets were collected by centrifugation, washed in phosphate buffered saline (PBS), filtered through a 70 $\mu$m mesh (Millipore, Billerica, MA, http://www.emdmillipore.com/), and the SVF cell concentrations determined by automated Cell Countess (Invitrogen, Carlsbad, CA, https://www.thermofisher.com/us/en/home/brands/invitrogen.html) count. The 1° SVF cells were suspended in Stromal Medium (DMEM/F-12 Ham's, 10% FBS [Hyclone, Logan, UT, http://www.hyclone.com], 100 U penicillin/ 100 g streptomycin/0.25 g fungizone) at a density of 0.156 ml of tissue digest per square centimeter of surface area for expansion and culture to get GFP-Tg ASC, or resuspended at a final concentration of 1 × 106 nucleated cells per milliliter in PBS, in preparation for staining.

SVF Cell Initial Immunophenotype and Subfractionation

Cell suspensions were incubated with antibodies against the cell surface antigens listed in Supporting Information Table at room temperature (RT) for 30 minutes, protected from light. After two washes with PBS, flow cytometric analysis was performed using a Beckman-Coulter Galios flow cytometer (BD Biosciences, San Jose, CA, www.bdbiosciences.com). The immunophenotype and relative subpopulations within the GFP-Tg SVF cells were determined out to passage 2 of plastic adherent culture using fluorochrome-conjugated monoclonal antibodies detecting the following panel of endothelial, hematopoietic, mesenchymal, and stem cell-associated antigens using the scheme provided in Supporting Information Table.

SVF Cell Selection

Two studies were performed that utilized GFP-Tg cells from GFP-Tg C57BL/6 mice (Figure 8). These include serial transplantation of GFP-Tg unfractionated SVF cells, and serial transplantation of live-cell sorted, culture expanded GFP-Tg ASC subpopulations. For the first study, GFP-Tg SVF cells were selected by flow sorting for the GFP-Tg population, and unfractionated GFP-Tg SVF cells were immediately loaded onto silk scaffolds for GFP-Tg SVF serial transplantation in non-GFP-Tg mice. For the second study, the GFP-Tg CD146-SVF subpopulation was selected and either plated as: (a) unfractionated controls, or sorted based on (b) CD29 positivity, and (c) CD34 positivity. The culture-expanded populations (a-c) were immunophenotyped, and loaded onto silk scaffolds for GFP-Tg ASC serial transplantation (ASC serial transplantation studies; see HexaFluoroIsoPropanol Silk Scaffold Loading, Culture, and Implantation below).

ASC Culture Expansion

Live cell sorting of GFP-Tg 1° SVF cells was performed using a BD Biosciences fluorescence-activated cell sorter (FACS) Beckman-Coulter Galios flow cytometer with two lasers and eight detectors running Galios acquisition software (BD Biosciences, San Jose, CA). All selected fluorochromes remained compatible with the continued use of the transgenic GFP for cell sorting purposes. Lineage depletion was performed using a cocktail of eFluor-conjugated antibodies detecting murine (mu) CD3, CD45R, CD116, LyG6, Ter-119 (eBioscience; 88-7772). Live SVF cell selection was performed with APC conjugated anti-muCD29 (17-0291) and PE conjugated anti-muCD34 (56-0341). ASC were isolated following plastic adherent culture expansion and using phenotypic and functional criterion established by International Federation of Adipose Therapeutics (IFATS) and the ISCT. Briefly, passage 0° (P0) ASCs were subjected to trypsinization with 5 ml 0.25% trypsin (Life Technologies, Grand Isle, NY) for 5 minutes. Trypsin digestion was stopped by the addition of an equal amount of ASC culture medium. P0 ASCs were counted using trypan blue dye exclusion, and passaged in new T175 flasks at a density of $0.4 \times 10^3$ cells per square centimeter. Cells were cultures until they reached 70% confluency. Populations were culture expanded to P2 as (a) unfractionated as controls or subfractionated into (b) a CD29+ enriched population (Lin-CD29+ CD146-) or (c) a CD34+ enriched population (Lin- CD34+ CD146-). The (a) unfractionated controls, (b) CD29+ enriched ASC populations, and (c) CD34+ enriched ASC populations were individually seeded in flasks and culture expanded according to our published and validated methods. After sorting for CD29, $95.05 \pm 2\%$ of the cells expressed CD29 and GFP. These SVF subpopulations were immediately plated, expanded, and passaged in order to get a sizeable population for seeding. Please note that the cells (CD29+ and CD34+ subpopulations) were selected separately, and were not sorted based on coexpression of both CD29 and CD34. In addition, the initial CD34 sorted subpopulation ($99.85 \pm 4\%$ positive for both GFP and CD34) included other SVF precursor populations that co-express CD34, and would be eliminated during plastic adherent expansion and selection of the adherent ASC populations; therefore, the initial sorted CD34 expression in SVF cells would be higher than that of culture-expanded CD34 selected ASCs, which have potential to lose the expression of the surface marker as a function of the adherence and proliferative processes. At passage 2 (P2), an aliquot of the ASC ($1 \times 10^5$ ASC) from each SVF fraction was characterized by analytical flow cytometry for GFP positivity and the antigens CD29, CD31, CD34, CD45, CD73, CD90, CD105, CD146, and Sca-1 to monitor changes in profile due to expansion. In parallel, the live P2 GFP-Tg ASC were evaluated for proliferation, colony formation, and adipogenic differentiation in vitro or for implantation studies.

HexaFluoroIsoPropanol Silk Scaffold Loading, Culture, and Implantation

The HexaFluoroIsoPropanol (HFIP) silk scaffolds obtained from the Tissue Engineering Resource Center, (Tufts University, Medford, MA, http://ase.tufts.edu/terc/) University were prewet overnight at 4°C in Stromal Medium (Dulbecco's modified Eagle'sHam's F-12 medium supplemented with 10% fetal bovine serum and 1% penicillin/streptomycin). The scaffolds were used in studies with unfractionated SVF cells, and with studies using fractionated passage 2 (P2) ASC. For the SVF study, the freshly isolated GFP-Tg iWAT 1° SVF cells were loaded by pipet in two aliquots of 50 μl each

containing a total of $2.5 \times 10^5$ cells to opposite faces of a cylindrical HFIP silk scaffold as per our published methods [33]. For the ASC study, a sample of freshly isolated GFP-Tg iWAT 1° SVF cells was directly plated (unfractionated), or sorted based on CD146- CD29+ or CD146-/CD34+ expression. The subfractions were plated and cultured until P2. The P2 ASC derived from unfractionated SVF controls, CD146- CD29+ enriched populations, and CD146- CD34+ enriched populations were loaded by pipet in two aliquots of 50 $\mu$l each containing a total of $2.5 \times 10^5$ cells to opposite faces of a cylindrical HFIP silk scaffold.

For both studies, the scaffolds were transferred to a humidified 37°C, 5% CO2 incubator, and rotated every 15 minutes over a 2-hour period. Following the addition of 5 ml of Stromal Medium, each scaffold was immediately implanted into 8-week male nontransgenic C57Bl/6 mice. A set of representative scaffolds (1° SVF cell or cultured ASC population) were retained, fresh frozen in optimal cutting temperature compound (OCT), and stored for use as positive controls. The remaining individual scaffolds loaded with SVF cells or ASC (n = 20 for each subpopulation) were implanted bilaterally into a dorsal subcutaneous pouch created in C57Bl/6 mice according to standard veterinary practices under an approved IACUC protocol (Protocol #4302). Each transplanted mouse was implanted with a randomized combination of empty scaffolds (control), those seeded with SVF cells, or P2 ASC that were unfractionated controls, CD29+ enriched population (CD29+ CD146-), or CD34+ enriched population (CD34+ CD146-).

Proliferation Assay

Immediately after cell seeding, the relative number of metabolically active stem cells within each seeded scaffold was determined by the AlamarBlue™ assay according to the manufacturer's instructions. Seeded scaffolds were incubated in Stromal Medium supplemented with 10% Alamar Blue reagent for 2 hours at 37°C with 5% $CO_2$. Aliquots (100 $\mu$l) of the culture medium were transferred to 96-well plates and quantified for fluorescence intensity with a microtiter plate reader (Fluostar Optima) using an excitation wavelength of 560 nm and an emission wavelength of 590 nm. Nonseeded scaffolds and tissue culture wells were also maintained in culture medium as above and were analyzed similarly as blank controls to adjust for background fluorescence. Scaffolds were then weighed and the relative cell numbers were calculated as the degree of their relative fluorescence intensity (RFU) per milligram of scaffold wet weight as previously described.

Adipogenic Differentiation

Adipogenic differentiation of culture-expanded and scaffold-seeded non-GFP-Tg ASC or GFP-Tg ASC was performed over a 15-day period as previously described. Briefly, cultured ASC were grown in Stromal Medium (Dulbecco's modified Eagle's-Ham's F-12 medium supplemented with 10% fetal bovine serum, and 1% penicillin/streptomycin). ASC were then trypsinized and plated in 24-well plates in ASC culture media at $3 \times 10^4$ cells per square centimeter for 24 hours to allow attachment. On day 1 (24 hours after plating), the medium was removed and cells were incubated for 3 days in adipogenic differentiation medium (Dulbecco's modified Eagle's-Ham's F-12 medium supplemented with 10% fetal bovine serum, 15 mM HEPES [pH 7.4], biotin [33 $\mu$M], pantothenate [17 $\mu$M, Sigma], human recombinant insulin [100 nM, Boehringer Mannheim], dexamethasone [1 $\mu$M], 1-methyl-3-isobutylxanthine [IBMX; 0.25 mM], and rosiglitazone [1 $\mu$M]). For the remaining 9 days of the adipocyte differentiation maintenance period, the medium was removed every 3 days and replaced with the same medium that did not contain IBMX and rosiglitazone (maintenance medium).

Adipogenesis of Scaffold-Seeded Cells

For scaffold-seeded GFP-Tg SVF cells and GFP-Tg ASC, one day prior to seeding, scaffolds were autoclaved and presoaked in adipogenic medium in 37°C, 5% CO2, 95% relative humidity. GFP-Tg ASC were then trypsinized and plated loaded onto presoaked scaffolds in ASC adipogenic differentiation media by pipet in two aliquots of 50 $\mu$l each containing a total of $2.5 \times 10^5$ cells to opposite faces of a cylindrical HFIP silk scaffold as per published methods. The cells were incubated for 3 days in adipogenic differentiation medium (Dulbecco's modified Eagle's-Ham's F-12 medium supplemented with 10% fetal bovine serum, 15 mM HEPES [pH 7.4], biotin [33 $\mu$M], pantothenate [17 $\mu$M, Sigma], human recombinant insulin [100 nM, Boehringer Mannheim], dexamethasone [1 $\mu$M], 1-methyl-3-isobutylxanthine [IBMX; 0.25 mM], and rosiglitazone [1 $\mu$M]). For the remaining 9 days of the adipocyte differentiation maintenance period, the medium was removed every 3 days and replaced with the same medium that did not contain IBMX and rosiglitazone (maintenance medium). Similarly, control cultures were maintained in parallel in the absence of adipogenic stimulants. All seeded and nonseeded (control) scaffolds were cultivated in a humidified incubator at 37°C with 5% $CO_2$. Following the in vitro cultivation period, seeded scaffolds were evaluated for their extent of adipogenesis.

Intracytoplasmic Lipid Quantification

For plastic adherent cultured ASC, lipid formation was assessed by incorporation of Oil-Red-O (ORO) (Sigma-Aldrich) into monolayers of GFP-Tg ASC cultured in adipocyte differentiation medium for 12 days. Quantitation of ORO incorporation was performed. Briefly, 0.5% (w/v) ORO was prepared in ethanol. Three parts ORO and two parts PBS were then mixed to make a working solution. Monolayers of GFP-Tg ASC cultured in 12-well plates were rinsed three times with PBS and subsequently fixed in 10% (v/v) formalin (Sigma-Aldrich) for 15 minutes. The monolayers were then rinsed three times with PBS and then incubated in ORO working solution for 45 minutes at RT. Following aspiration of unincorporated ORO, monolayers were rinsed four times with PBS. Stained monolayers were visualized with phase contrast microscopy (Eclipse 800, Nikon, Tokyo, Japan, http://www.nikon.com/). Incorporated ORO was extracted by incubating stained monolayers in 100% isopropanol for 10 minutes. The absorbance at 510 nm of each aliquot was then measured using a 96-well plate reader.

For scaffold-loaded ASC, accumulated lipid was also visualized using ORO staining. Scaffolds were fixed in 2 M sucrose overnight for histology. The scaffolds were embedded in OCT medium, frozen over dry ice, and stored at -80°C. Frozen scaffolds were then sectioned (7-$\mu$m sections) using a cryostat and stained. For ORO staining, frozen sections were fixed in 10% buffered neutral formalin for 15 minutes, and rinsed with distilled water. ORO working solution was placed onto frozen sections and incubated for 20 minutes. Slides were rinsed with distilled water, and counterstained with 4',6-diamidino-2-phenylindole (DAPI) according to manufacturer's protocol. Stained sections were covered using glass cover slips. Images were taken using phase contrast microscopy.

Serial Transplantation

The transplanted animals were maintained for 6-8 weeks, euthanized, and the scaffolds were removed using aseptic dissection techniques. Prior in vivo studies have demonstrated that 4-6 weeks is a sufficient period to allow for human ASC adipogenesis in transplanted silk scaffolds. We observed a surge in GFP-Tg cell expansion and differentiation by confocal microscopy following 6 weeks. Therefore, our studies were carried out to week 6. Sets of n = 2 scaffolds were fresh frozen in OCT for microscopic analyses. The remaining n = 18 scaffolds were subjected to collagenase digestion for 1 hour in PBS supplemented with 0.1% collagenase type I (Worthington Biochemicals, Brunswich, Lakewood, NJ, http://www.worthington-biochem.com), 1% bovine serum albumin, and 2 mM CaCl$_2$ for 60 minutes at 37°C with intermittent shaking. The 2° (secondary implanted) SVF cells were isolated as described above, under HFIP Silk Scaffold.

Loading, Culture, and Implantation

The frequency of GFP-Tg cells in the 2° SVF as well as their expression of associated antigens (panel of monoclonal antibodies (mAbs) above) were determined by analytical flow cytometry using a sample of each grouping. The remaining sorted 2° SVF cells from the original cohorts were loaded onto freshly prepared HFIP silk scaffolds by repeating the same process above. After incubation, the scaffolds were loaded with the 2° SVF cells and were implanted bilaterally into C57Bl/6 mice for a 6-week period. At that time, the scaffold harvest and analysis was repeated to yield a 3° SVF cell population. Due to cell harvest constraints, the scaffold harvest and analysis was terminated following 3° SVF cell populations, and 2° ASC populations. As a positive control, intact inguinal depots from C57Bl/6 (UBC-GFP) mice were serially transplanted to congenic wild-type recipients for equivalent periods of time. Additionally, insulin sensitivity within the removed silk scaffold constructs was determined using our published in vitro glucose uptake and lipolysis assays to insure functionality. It should be noted that for the present study time of engraftment was measured between the date of surgical implantation of the scaffold (with or without cells) and the date when it was surgically removed from the animal. For negative control groups, tissue was harvested from age and gender compatible non-GFP-Tg C57BL6 mice.

Hemoglobin Saturation Measurement

The Zenascope PC1 spectrometer (Zenalux, Durham NC, http://www.zenalux.com) employs a broadband halogen light source (probe) to illuminate the target tissue. The Zenascope allows measurements of protein levels in live animals, in the absence of invasive techniques. A bifurcated fiber optic probe with stainless steel jacketing and terminating in a 0.25" diameter stainless steel rigid common end is applied to the surface of the tissue, delivers light to the target tissue and collects the reflected optical signal, and the optical properties (wavelength-dependent absorption and scattering coefficients) of the tissue are quantitatively extracted from the measured reflectance spectra over the wavelength range 500-650 nm. From the extracted absorption coefficient, the portable and standardized measurement hardware achieves rapid, real-time quantitative analysis of oxyhemoglobin (HbO2), deoxyhemoglobin (dHb), total hemoglobin concentration (Hb, defined as HbO2 + dHb), and hemoglobin oxygen saturation (sO2, defined as the ratio HbO2/Hb). The optical reduced scattering coefficient was also measured and reported by the instrument, which allows measurements of tissue chromophores to be quantified accurately and independently of changes in tissue scattering. Integration times were automatically set by the system software for each measurement to maximize reflectance signal while staying within the linear response range of the detector, and typically ranged from 100 to 200 ms. Reflectance calibration of the probe using a 99% reflectance standard was performed prior to all experiments and periodically during the procedures.

ImageJ Explant Analyses

For reporting on ASC or SVF enhancement engraftment time, three representative images of whole explants that were removed from each cohort were converted to TIF files and exported to ImageJ for analyses. Images from unseeded scaffold explants (control) were quantified using (ImageJ Version 1.46 software, NIH). Pixel values were from the entire tissue explant, not sectioned images. Threshold values were established from subtracting background pixel count using the control. Relative mean pixel counts from groups of tissue sections were averaged and the standard deviation was calculated.

Cryo-Scanning Electron Microscopy

Cryo-scanning electron microscopy was conducted on scaffolds that were seeded with GFP-Tg ASC following 24-hour cell culture. Cryo-SEM structure research was done with Gatan 2500 alto Cryo-system and Hitachi S-4800 scanning electron microscopy (SEM). The tissue was cut to $7 \times 5 \times 5$ (mm) and laid about 5 mm high above a cryogenic SEM sample holder surface, clamped and glued into the holder, and then frozen in slushed liquid nitrogen at approximately -210°C for about 30 seconds. The frozen tissue was transferred in vacuum to the prechamber attached to the SEM and then fractured and sublimed for 5 minutes at -95°C. After coating 88 seconds at -130°C with Pt/Pd, the sample was moved to the SEM and observed at 3kV at -130°C.

Histochemistry

Fresh frozen tissue samples were sectioned, stained with a lipophilic fluorescent dye (Bodipy), and evaluated by confocal fluorescent microscopy for the colocalization of GFP and lipid signals.

Quantitative Polymerase Chain Reaction (qPCR) for GFP Gene Expression

Total DNA was isolated from removed tissue engineered fat pads or positive or negative controls using the Qiagen FFPE tissue prep Mini Kit (Qiagen Inc., Valencia, CA, http://www.qiagen.com) according to the manufacturer's specifications. Polymerase chain reactions were performed with approximately 500 ng of total DNA using the SYBR green real-time PCR DNA binding dye. Reaction mix was incubated with Oligonucleotide primer sets specific for GFP (forward: 5'-TCGCCTACCAGCTCATGCATAACA-3'; reverse: 5'-TGAAGCTCTTCCAGGTGTCAACGA-3') in a real-time thermal detection system (Bio-Rad Laboratories, Hercules, CA, http://www.bio-rad.com). All results were normalized relative to glyceraldehyde 3' phosphate dehydrogenase (GAPDH) expression control.

Statistics

All studies were repeated at least in triplicate and values are reported as the mean $\pm$ standard deviation (SD). Individual pairs were compared using the student T test while larger groups were analyzed by Analysis of Variance. Findings were defined as significant with minimum p values $\leq$.05.

Results

Isolation and Characterization of Cells Utilized

Figure 1A provides the representative subpopulations of cells detected within GFP-SVF of iWAT isolated from 8 to 12 week male mice. Initial SVF yields averaged 3-5 $\times$ 10$^5$ 1° (primary) SVF cells per milliliter of adipose tissue. This is similar to reported values in the literature. The three largest subpopulations based on surface immunophenotype were the preadipocytes, (24.7 $\pm$ 6.9%; CD14-, CD36+), the ASC-like (20 $\pm$ 10.7%; CD146-, CD34+, CD90+), and the leukocytes (19.4 $\pm$ 9.7%; CD34-, CD45+). Of note, the pelleted SVF cell population excludes floating mature adipocytes (estimated to be about 30% of the total population) due to the process of collagenase type I tissue digestion and isolation by differential centrifugation. The initial SVF fraction was first sorted for the CD146- subpopulation (80.5 $\pm$ 5%). This served as the initial cohort of fractionated SVF cells. Other cohorts were selected based on sorting for CD34 and CD29 surface antigen expression: (b) CD29+ (18.2 $\pm$ 8.5% of the CD146- population), and (c) CD34+ (21.4 $\pm$ 12.8% of the CD146-population) SVF cells. The flow cytometry sorting protocol is given in Figure 2. Characteristics of these cohorts [(a) total CD146- SVF cells, (b) CD146-, CD29+ enriched cells, and (c) CD146-, CD34+ enriched cells] were determined based on immunophenotype of plastic adherent cell culture, proliferation, colony formation, and adipogenic potential out to passage 2 (P2). Similar to other published reports, stromal markers (CD29) increased in expression, and endothelial (CD31, CD45) and hematopoietic markers (CD34, CD11b) decreased in expression over passage. Sca-1 expression averaged $61.6 \pm 10.7\%$ within unsorted SVF populations and increased over passage (Fig. 2A). CD29 and CD34 enrichment resulted in lower Sca-1 expression (CD29; $13.8 \pm 2.4\%$, CD34, $13.7 \pm 0.9\%$). CD34+ enriched subpopulations exhibited significantly higher proliferative and adipogenic potential than CD29+ enriched subpopulations (Fig. 2B). Further, CD29+ subpopulations had slower doubling times and lower intracytoplasmic lipid accumulation than the unsorted populations (Fig. 2C, 2D).

SVF and ASC Seeding on Scaffolds

GFP-ASC in vitro seeding and proliferative properties were investigated following seeding on HFIP-based silk scaffolds. Confocal microscopy was performed on seeded and unseeded control scaffolds after 24 hours of incubation (Figure 9). GFP detection and Alamar Blue staining revealed a preference of cell localization on the edges of the silk scaffold honeycomb-like structures (Fig. 9B, bright field and Fig. 9D, scanning electron microscopy). Cells remained on these edges, commenced differentiation (Fig. 9C) and proliferation (Fig. 9D, 9E). Quantitation of Alamar Blue (Fig. 9D) cytosolic and DAPI (Fig. 9E) nuclear positivity revealed the cells were present 2 hours post seeding (18 hours; $16 \pm 2$ cells; 2 hours, $11 \pm 8$ cells; Fig. 9E), but adhered to the scaffold and exhibited measurable metabolic activity by RFU 18 hours post seeding (18 hours, 36 RFU; 2 hours, 0.001 RFU; Fig. 9E).

SVF Cells Enhance Engraftment and Generate Adipose In Vivo

Unfractionated SVF cells were monitored for growth and adipogenesis using a 6-week time course. Photographic analyses revealed that the presence of SVF cells accelerated engraftment by week 1(Fig. 3A). Measurements of percent hemoglobin saturation (%Hb; Fig. 3B) also supported the observed SVF cell-mediated enhanced vascularization. It should be noted that hemoglobin measurements were taken on the skin surface of live mice, and that the measurement of saturation assesses all tissues, not simply the arterial blood supply. By week 2 and beyond, no significant difference in engraftment was observed, both visually and by quantification of explant images using ImageJ (Fig. 3C). This supported published data suggesting the vascular and supportive nature of the scaffolds to promote adipogenesis in vivo. Measurements of scaffold mass (Fig. 3D) and total SVF recovered (Fig. 3E) from seeded scaffolds were significantly higher than unseeded controls during week 1. SVF cell count and scaffold mass reached a maximum at week 2, and decreased by week 4 (Panels 3D, 3E).

Unsorted GFP-SVF Cells Detected Following Serial Transplantation

Weekly detection of unsorted GFP-SVF within initial implants from weeks one to six (Fig. 4A) revealed that GFP-SVF cells were not only persisting within scaffolds, but also proliferating and differentiating as early as week 1 following implantation. Confocal microscopy images are displayed for weeks 1, 4, 5, and 6 of initial SVF explants (Fig. 4A). GFP-Tg SVF cells were detected within explants from initial cohorts (T0 implants), as well as initial serial (T1 implants) and secondary serial (T2 implants; Fig. 4B) transplants. Flow cytometry-based detection of GFP-Tg SVF cells was consistent with confocal microscopy observations (Fig. 4C). Initial implants resulted in 19% GFP antigen positivity via flow cytometry at week 4 while T1 and T2 implants resulted in 16.2% and 13.1% GFP positivity, respectively, at the same time points relative to implantation (Fig. 4C). Quantification of GFP, BODIPY lipophilic dye staining (to demonstrate the adipogenic nature of the constructs), and the nuclear DAPI stain colocalization was performed using ImageJ. Quantification demonstrated a 50% increase in GFP-Tg SVF cell percentage by week 6 following T0 implants, compared to week 1 (Fig. 4D). It is important to note that the flow cytometry data reported in Figure 4D is conducted on the SVF cells that have been isolated from the tissue reported in Figure 4C. Adipose tissue contains mature adipocytes and other cells that would not survive the tissue digestion procedure, and would not be reflected in the flow cytometry numbers. Figure 4D, therefore, does not reflect all cells in the tissue sample. GFP expression within removed explants was further investigated based on protein and DNA levels via flow cytometry and qPCR, respectively (Fig. 4E, 4F). Weekly detection of percent GFP-Tg cells via flow cytometry revealed an increase in expression from 3.8% in week 2 to 16% by week 4 following T0 implants (Fig. 4E). GFP DNA expression was enriched more than 200-fold (unseeded scaffold implants in nontransgenic mice) following T2 implants (Fig. 4F). The surface immunophenotype of cells from the T2 implant population was also compared to GFP-Tg controls and unseeded scaffold controls following 6 weeks (Fig. 4G). The T2 scaffolds displayed $36.3 \pm 2.4\%$ GFP positivity, $67.8 \pm 4.1\%$ CD29 positivity, and $52.4 \pm 2.4\%$ CD45 positivity (Fig. 4G).

CD34-Enriched GFP-Tg ASC Enhance Engraftment and Generate Functional Adipose In Vivo

GFP-Tg SVF cells were isolated and grouped as unfractionated cells, or sorted as CD146- CD29+ enriched, or CD146- CD34+ enriched groupings. These cells were culture expanded to P2 GFP-Tg ASC, seeded on silk scaffolds, and implanted into nontransgenic mice until T0 serial transplant. No visual difference in fat depot size was observed at week 6 of implant removal (Fig. 5A, 5B). Measurements of percent hemoglobin (%Hb) saturation and explant engraftment were investigated. Implants seeded with both CD29- and CD34-enriched GFP-Tg ASC exhibited higher %Hb saturation following 1 week of implantation compared to both unseeded implants and implants seeded with unsorted ASC control (Fig. 5C). Further, CD34-enriched implants exhibited significantly higher %Hb saturation than CD29-enriched implants (Unseeded ASC: control, $37.6 \pm 9.9\%$; CD34-enriched, $68.1 \pm 5.3\%$; CD29-enriched, $45 \pm 10.8\%$; Fig. 5C). This was observed visually and quantitatively using ImageJ software analyses of photomicrographs (Fig. 5D). Measurements of explant mass and mass of the surrounding fat (Fig. 5E) revealed that the engineered constructs originating from CD34-enriched implants were significantly more dense than CD29-enriched and unsorted GFP-Tg ASC, as well as unseeded controls (Fig. 5E). Total SVF recovered (Fig. 5F) from CD34-enriched ASC-seeded scaffolds were also significantly higher than CD29-enriched, unsorted ASC, and unseeded controls during week 1 (Fig. 5F). Further investigation of tissue engineered fat functionality revealed CD34-enriched constructs secreted glycerol levels comparable to unsorted ASC constructs, whereas CD29-enriched constructs secreted significantly lower levels of glycerol (Fig. 5G). No significant difference was observed in glucose uptake between cohorts (Fig. 5H).

CD146- CD34+/CD29+ GFP-Tg ASC Detected Following Serial Transplantation

GFP-Tg ASC were detected within 6-week explants from initial cohorts (T0 implants), as well as initial serial (T1 implants; Fig. 6A). Tissue implants were infiltrated with blood vessels, which supports the earlier observation of tissue engraftment with GFP-Tg SVF and GFP-Tg ASC seeded scaffolds. Flow cytometry-based detection of freshly isolated GFP-Tg cells supported confocal microscopy observations of GFP positivity following week 6 of implantation (Fig. 6C, 6D). Initial CD34-enriched constructs resulted in detection of 29.4% GFP antigen positivity, compared to CD29-enriched constructs, which resulted in 10.9% GFP positivity via flow cytometry (Fig. 6C). CD34-enriched and CD29-enriched T1 implants resulted in 20.4% and 8.4% GFP positivity, respectively (Fig. 6D). Quantification of GFP, BODIPY lipophilic dye, and DAPI colocalization staining was performed using ImageJ. GFP expression within explants was further investigated based on DNA and protein levels via qPCR and flow cytometry, respectively (Fig. 6E-6G). GFP DNA detected was 15-fold higher in CD34-enriched constructs compared to CD29-enriched constructs, and more than 10-fold higher than unsorted ASC constructs following T1 implants (Fig. 6E). Detection of percent GFP-Tg cells via flow cytometry supported a significant difference in expression of GFP-Tg cells from CD34-enriched constructs compared to CD29-enriched constructs following 8 week T0 implants (CD29, $9.8 \pm 1.1$; CD34, $24.7 \pm 6.5$; Fig. 6F) and T1 implants (CD29, $5.4 \pm 1.2$; CD34, $17.5 \pm 1.4$; Fig. 6G).

CD146- CD34+-Enriched Constructs Generate Functional GFP+ Fat In Vivo

Adipose surrounding removed 8-week serial implants from GFP-Tg SVF seeded, unsorted GFP-Tg ASC, GFP-Tg CD34-enriched ASC, GFP-Tg CD29-enriched ASC, and GFP positive and negative controls, were removed and analyzed for GFP positivity (Fig. 7A-7C) and functionality (Fig. 7D, 7E). Confocal microscopy images of BODIPY stained removed adipose surrounding 8-week constructs revealed a significant increase in percent GFP-Tg adipocytes in CD34-enriched cohorts compared to CD29-enriched, unsorted ASC, and SVF-seeded constructs (Fig. 7A, 7B). Quantification of images using ImageJ supported visual observations (SVF, $2.5 \pm 1.7\%$; unsorted GFP-Tg ASC, $15.5 \pm 0.2\%$; CD29-enriched, $6.1 \pm 7.0\%$; CD34-enriched, $31.3 \pm 0.4\%$; Fig. 7C). Further investigation of tissue engineered fat functionality revealed CD34-enriched constructs secreted glycerol levels comparable to unsorted ASC constructs whereas CD29-enriched constructs secreted significantly lower levels of glycerol (CD34, $70 \pm 16.1 \mu M$; unsorted ASC, $75 \pm 22.1 \mu M$; CD29, $40 \pm 14.6 \mu M$; Fig. 7D). Similar to removed constructs in Figure 5, no significant difference was observed in glucose uptake between cohorts (Fig. 7E).

Discussion

The present study transplanted, freshly isolated GFP-Tg SVF cells using 3D silk matrices into non-GFP-Tg transgenic syngeneic murine hosts successfully over one initial, and two serial implants (2° implants). Initial time-based implant removal studies demonstrated GFP-Tg SVF cell survival, proliferation, and differentiation, leading to the formation of metabolically functional adipose tissue. Time-based GFP-Tg SVF experiments also revealed SVF cell ability to enhance engraftment and to reduce engraftment time. Expanded, plastic-adherent CD146- CD29+ and CD146- CD34+ P2 GFP-Tg ASC populations were seeded on 3D matrices, and implanted into non-GFP-Tg transgenic hosts successfully for an initial and serial implant (1° implants). To our knowledge, we are among the first to report the ability of specific subpopulations of nonpericytic ASC within the stromal vascular compartment to generate functional adipose over serial transplants.

Mauney et al. reported on the ability of culture-expanded, cryopreserved ASC that had not been selected or enriched by flow cytometry to generate fat pads in rats using engineered scaffolds. The objectives of their study included comparing biomaterials derived from silk fibroin prepared by aqueous (AB) and organic (HFIP) solvent-based processes, along with collagen and poly-lactic acid-based scaffolds, for their utility in adipose tissue engineering strategies. In contrast, the present disclosure provides 1) a different, more heterogeneous stromal vascular population including surface-antigen-selected subpopulations and other cells within the SVF that more effectively promote and retain adipogenesis and fat engraftment, 2) serial transplantation as a model for explaining the behavior of the cells in the disclosure, and 3) an extended *in vitro* culture.

Due to the limitations of mouse tissue volume, ASC used in the present study were pooled from multiple mouse donors, and starting sample sizes for serial transplants were n = 20. A caveat within this study is the usage of CD34-enriched and CD29-enriched ASC populations that were expanded from freshly isolated mouse GFP-SVF; not CD34-exclusive and CD29-exclusive populations. Due to the rarity of CD29+/CD34+ double positive and CD29-/CD34-double negative subpopulations within the freshly isolated SVF subfraction, the groupings in this study contained culture-expanded CD34+ cells without consideration of CD29 coexpression. The same selection was used for CD29, without consideration of CD34 coexpression. Therefore, a subpopulation within the CD34+ GFP-ASC population also coexpressed CD29 and other cell surface and intracellular stromal markers that are involved in anchorage, proliferation, migration, and adipogenic regulation. CD34+ populations within freshly isolated murine SVF cells averaged 21.4% in the unsorted population; however, the CD34+-enriched population in the SVF maintained an average of 33% positivity after two passages in culture. The percentage CD34+ GFP-Tg ASC in this study are similar to published reports on CD34 positivity within human SVF. The human CD34 positive populations were demonstrated to possess enhanced proliferative and adipogenic potential in vitro. A strong correlation was also identified between CD34+ ASC yield and xenograft adipose tissue volume retention, suggesting that concentration of the CD34+ progenitor can predict human fat graft survival in clinical settings. Future experiments will extend the study to a more humanized system, initially by examining the behavior of sorted human cells in an immunodeficient murine model. These experiments will also allow for larger initial sample sizes that are greater than n = 20.

The current literature refers to cells that aid in fat grafting survival using the terms, "adipose stromal" cell and "perivascular stem" cell interchangeably. Traditionally, perivascular stem cells are identified based on an immunophenotype of CD45-, CD146+, and CD34-. The results from CD146- CD34+-enriched ASC serial implants indicate the presence of a nonpericytic ASC subpopulation within SVF that is capable of generating functional fat pads in mice over successive transplants. These findings are supported by in vivo cell fate/mapping studies where tissue resident stem cells in neonatal mice were labeled with bromo-deoxyuridine (BrdU). Eight weeks later, histological analyses of inguinal adipose depots determined that the BrdU label retaining stem cells were found in the perivascular space (consistent with a pericytic subpopulation) and at a substantial distance from the capillaries, in the vicinity of mature adipocytes (consistent with a nonpericytic population). Although the CD146- population represents the majority of the plastic-adherent population (80%) in this study, only 20% of these cells coexpress the progenitor cell antigen CD34. Nevertheless, the current results indicate that nonpericytic cells can serve as adipogenic stem cells *in vivo.*

Confocal fluorescence imaging measurements of %Hb saturation at weeks 1 and 4 demonstrated that the presence of GFP-Tg SVF or enriched GFP-Tg ASC accelerated scaffold engraftment as early as week 1 of implantation. Measurements of the SVF recovered from the explants and scaffold weights at weeks 1 and 2 supported these data. Current published reports suggest SVF and ASC encourage the enhancement of engraftment and acceleration of wound healing in a variety of applications, including both soft and hard tissue remodeling, and for the improvement of pathophysiological conditions [42]. Mesimaki et al. demonstrated the usage of expanded ASC for the enhancement of a maxillary flap with β-tricalcium phosphate and bone morphogenetic protein 2 (BMP2). Addition of the expanded ASC aided in the development of mature bone structures and vasculature. Yoshimura et al. reported high surgeon and patient satisfaction 12 months postoperatively in 55 patients receiving adipose tissue grafts augmented with SVF cells, a procedure termed "cell-assisted lipotransfer (CAL)," instead of prosthetic breast enhancement. More recently, Bura et al. have employed autologous human ASC to treat patients with critical ischemic limbs and have observed significant improvements in tissue oxygen delivery. Together, these human clinical studies lend credence to the findings in our murine preclinical model.

Confocal microscopy and flow cytometric analyses of 8-week serial implants indicated that CD34+-enriched subpopulations are capable of generating functional fat pads, similar to unsorted ASC implants. CD34+-enriched ASC were demonstrated to proliferate and undergo adipogenic differentiation at higher rates than CD29+-enriched and unsorted ASC in vitro. In vivo, higher percentages of SVF cells were recovered from CD34-enriched constructs. These explants averaged higher scaffold masses than CD29-enriched and unsorted ASC cohorts. Functional analyses of fat surrounding the scaffolds after 6 weeks revealed CD34-enriched explants exhibited higher glycerol secretion than CD29-enriched, unsorted ASC, and control unseeded implants. GFP-Tg SVF and -ASC were detected within surrounding adipose depots. BODIPY staining, glycerol uptake, and lipolysis analyses suggest these cells were proliferating, differentiating within the scaffold, and migrating locally as mature adipocytes or as preadipocytes. These data suggest that a subpopulation phenotypically found within the CD146⁻ CD34⁺-enriched ASC population generates functional fat pads over serial passages. These findings are consistent with studies by Philips et al. demonstrating that the functionality and volume retention of human adipose tissue xenografts correlated with the frequency of CD34⁺ cells in the donor tissue.

Conclusion

It remains challenging to distinguish cell categories between "stromal/progenitor" and "stem" cell. Nevertheless, the current serial transplant findings suggest that the inguinal WAT of adult mice contains a flow cytometry, sortable subpopulation of cells capable of contributing to the regeneration and serial transplantation of a functional adipose tissue depot. These findings confirm and extend earlier studies by Rodeheffer et al. examining the ability of flow cytometry sorted adipose-derived cells to regenerate adipose depots in lipodystrophic mice. Additionally, these outcomes further validate the utility of autologous adipose-derived cells for functional tissue engineering. The work also extends prior studies characterizing the immunophenotype and pericyctic properties of isolated adipocyte progenitor cells.

## Example 3. Experimental for Human Platelet Lysates (hPL) Gel Preparation

When hPL and DMEM F12 combine, and are warmed to 37°C, the product is a thermos-responsive bioscaffold that is conducive to extended (1-2 month) 3-D culture and/or in vivo implantation via subcutaneous injections.

Methods

Preparation of hPL

Perform all steps within a biological safety cabinet unless otherwise indicated. Obtain desired amount concentrated expired human platelets in 15mL or 50mL conical tube. If not using immediately for 3-D cell culture, store at -20°C before lysate preparation by three freeze/thawing cycles. Perform 3 freeze/thaw cycles to lyse platelets by osmosis. This will release maximum yield of growth factors for cell cultures. Centrifuge 8,000 x g for 20 mins. Remove the solid (white) top layer of human platelets by aspiration.

hPL supplemented media preparation

Prepare hPL supplemented media by adding 7.5% (v/v) hPL to 1% antibiotic/antimycotic and 91.5% Dulbecco's Modified Essential Medium or other related cell culture medium containing necessary nutrients, amino acids, and growth factors for cell culture. After mixing, do not warm in water bath. Maintain at room temperature.

hPL matrix device activation

Activate gelation (formation of a thick matrix) conducive to cell secretion of extracellular matrix, proliferation, and differentiation by combining prepared hPL with cell population or mixture of cell populations. After combining cells and hPL, add plate to incubator for 20 mins for activation of gelation. Proceed to proliferation, differentiation, implantation into mice, or other measured endpoints.

## Example 4. Fat on a Chip Technology

Except for skin cancer, breast cancer is the most frequent tumor among U.S. women where it remains the second leading cause of cancer-related death. The risk of breast cancer death is particularly high in African American women where the disease is often associated with a worse prognosis attributed to dietary/nutritional, economic disparities, genetic, health access, and obesity related factors. While patient derived xenografts (PDX) models maintained in immunodeficient mice hold promise as a pre-clinical model for development of novel breast cancer therapies, there are limited numbers of PDX breast cancers commercially available for contract research and oncological pharmacology development. The inventors, a minority and woman owned biotech company, is uniquely positioned to access breast tumor specimens from the ethnically diverse patient demographic living in the greater metropolitan New Orleans area and neighboring southeastern Louisiana.

The present inventors have sought to build on certain information including: (1) Human platelet lysates contain an abundance of growth factors in high concentration that enhance vascularity and have been reported to promote colony formation by primary breast tumor cells; (2) Stromal vascular fraction (SVF) cells isolated from subcutaneous adipose tissue contain lymphocytes, myelocytes, pericytes, endothelial progenitors, and pre-adipocytes necessary to develop a 3-dimensional adipose depot and; (3) Human adipose-derived stromal/stem cells (ASC) promote the proliferation, migration, and invasion of breast cancer in vitro and in vivo through adipokine (leptin, among others) secretion and related mechanisms. While decades of research using established human breast cancer cell lines has generated thousands of manuscripts, these two-dimensional culture systems often fail to accurately mimic the breast cancer microenvironment with respect to immune and stromal cell interactions. A relatively limited body of work using patient derived xenografts (PDX) has begun to address this unmet need for a patho-physiologically accurate and reproducible human disease model. Studies are hampered, in large part, due to the extended time required to establish human xenografts in mice and their low rate of engraftment. The inventors have developed innovative approaches addressing these limitations and needs.

Human SVF cells isolated from subcutaneous adipose tissue lipoaspirates have been cryopreserved and quality controlled based on flow cytometry characterization, colony unit formation, proliferation, and adipogenic and osteogenic differentiation (data not shown). The behavior of cryopreserved SVF cells has been evaluated in the 3-D mammalian platelet lysate culture system (Figures 16 and 17). The hSVF cells increased their proliferative capacity in the dose-dependent presence of mammalian platelet lysate relative to control medium containing 10% FBS (lot characterized and screened based on optimal adipose stromal/stem cell growth and adipogenesis). Furthermore, the hSVF cells increase their formation of spheroids with the presence of increasing concentrations of mammalian platelet lysate (Figure 16). In the absence of adipogenic inductive factors, the hSVF cells in mammalian platelet lysate containing scaffolds display the formation of vascular like networks over a 3-week culture period. With the added presence of adipogenic inductive agents, the hSVF cells accumulate lipid vacuoles, as detected by confocal microscopy and Bodipy staining (Figure 17).

Functionality has been validated by quantitative biochemical assessment of adipokines (adiponectin, leptin) secretion in white (WAT) adipose tissue engineered constructs. Adipokine secretion is enhanced in the 3D relative to 2D constructs (data not shown). Functionality has been validated further by quantitative physiological assessment of lipolysis, glucose uptake, and cellular heterogeneity based on flow cytometry (data not shown).

The hSVF cell/Mammalian platelet lysate scaffolds have been implanted in immunodeficient (nude $^{nu/nu}$) mice to create a physiological 'humanized fat pad' (Figure 18). These preliminary data validate the inventors' technical expertise in developing a novel, functional and commercially applicable "fat on a chip".

Adipose-Derived Cells Promote Breast Cancer Cell Line Proliferation: Studies by the PI and her collaborators have demonstrated that human adipose-derived cells promote the proliferation of breast cancer cell lines in vitro and in vivo. The body mass index of the adipose tissue donor contributes to the breast cancer cell growth. Obese, as opposed to lean, donor derived stromal cells enhance breast cancer cell proliferation via their increased secretion of the adipokine leptin. Thus, Mammalian platelet lysate, by promoting hSVF cells adipogenesis within the humanized "fat on a chip", will directly impact the engraftment and proliferation of breast tumor xenografts in immunodeficient mice.

Statistics: All experiments in Experiment 1 and Experiment 2 will be performed in triplicate animals with duplicate implants per animal. Quantitative results will be evaluated and reported as means $\pm$ standard deviation. Comparisons will be assessed between controls (tumor implants without SVF cells or scaffolds) and experimental conditions (tumor implants with SVF cells and/or scaffold) based on two tailed t-test, ANOVA, or Tukey (where Confidence Interval 95%, p-value < 0.05 is considered significant).

Hypothesis 1 and Experiment 1: That the human platelet lysate functions as well or better than Matrigel™ as scaffold for growth of breast cancer cell lines implanted into immunodeficient mice (NSG) in combination with human SVF cells.

Rationale: The oncology community has considerable interest in expanding the scope of PDX models for purposes of prognosis, diagnosis, and therapy, particularly in the context of health disparities facing African American patients. Major challenges are (a) the failure of 40% to 80% of PDX to successfully engraft in the immunodeficient host and (b) the lack of sensitive non-terminal methods to monitor the engraftment's success or failure in real time [10, 20]. Studies of colon cancer PDX models have demonstrated that inclusion of Matrigel™ with the primary tumor xenograft can significantly increase the incidence of a successful engraftment following implantation into immunodeficient mice. While Matrigel's exact mechanism remains unproven, its ability to enhance and accelerate tumor growth rate has been attributed to the contribution of growth factors and cytokines to the tumor's microenvironment. This is supported by the fact that Matrigel™ can promote breast cancer cell growth in vitro. The inventor's proprietary products (Mammalian platelet lysate and adipose-derived SVF cells) have unique features as a source of cytokines and growth factors and, furthermore, can create a 3-dimensional cellularized matrix upon incubation at body temperature. Additionally, Innogenomic's sensitive human cell detection methods permit early detection and quantitative assessment of tumor cell growth in live animals without requiring the euthanasia of the host. Experiment 1 will use one or more human breast cancer cell lines to establish proof of principle data documenting the ability of adipose cell and/or matrix to accelerate tumor growth in vivo.

Experimental Design:

Rigor and Transparency: The immunodeficient murine strains will be obtained directly from Jackson Laboratory, an NIH supported contract research facility that performs rigorous analysis and quality assurance of its murine strains. The established breast cancer cell lines will be sourced directly from the American Type Culture Collection, an NIH supported cell repository that performs rigorous analyses on its products. The inventor maintains de-identified anonymous records of the critical demographics (age, gender, body mass index, ethnicity) of the SVF cell donors. All cryopreserved vials of the company's cell products are entered into an updated database and the integrity of the storage vessel is monitored daily by the company's technical staff. The inventor performs routine quality assurance and quality control testing on its Mammalian platelet lysate products and SVF cells using in vitro cultures, flow cytometry, and differentiation assays. Because breast cancer occurrence in women exceeds that of men by nearly two orders of magnitude, Phase I studies will be performed using female NSG mice and hSVF cells isolated from female donors. Additional studies examining male mice and male adipose donors will be considered in Phase II only if recommended by the Study Section.

Methods: All studies will be performed under appropriately reviewed and approved IRB protocols (Western IRB or Ochsner IRB), patient consents (signed or waived), and IACUC protocols (Tulane University IACUC). SVF Cell Isolation and Characterization: Human SVF cells will be isolated from subcutaneous adipose tissue donated by healthy subjects undergoing elective lipoaspirates under a protocol by the inventor's Sciences partner, LaCell LLC. Tissue will be washed in PBS, digested with collagenase type 1, and SVF cells isolated by density centrifugation prior to cryopreservation as previously described. Initial studies will use hSVF cells pooled from n = 3 obese female donors with BMI > 30. As time and resources, permit, additional studies will be performed using hSVF cells pooled from n = 3 lean female donors with BMI < 25. At the time of thawing, the viability of the SVF cells will be assessed using a fluorescent based live/dead assay (LaCell LLC reagent).

hSVF Cell/Scaffold Adipogenesis: The hSVF cells ($10^6$) will be combined with 250 µl of 25% Matrigel or Mammalian platelet lysate, plated into individual wells of a 12 well plate, and cultured for one week in Stromal Medium™ (LaCell LLC). The SVF/scaffold medium will then be replaced with AdipoQual™ (LaCell LLC) for an additional week and the "fat on a chip" monitored by light microscopy for visualization of lipid vacuole accumulation.

Breast Cancer Cell Line In Vivo Implantation: Human breast cancer cell lines MCF7, ZR75, and/or T47D will be sourced directly from the American Type Culture Collection and expanded in vitro. Female NSG mice will be anesthetized, a midline incision will be made with scalpel, and bilateral pockets dissected subcutaneously over the dorsal flank by blunt dissection. Prior to implantation of 106 breast cancer cells into the bilateral dorsal flanks of immunodeficient female NSG mice, either as cells alone, cells with Matrigel or Mammalian platelet lysate alone, or with the addition of $10^6$ obese or lean hSVF cells as outlined in the Tables for Study 1 and Study 2 [6]. The rate of tumor growth will be assessed bi-weekly based on caliper measurements of the width and length of the tumor(s) as well as based on serum detection of circulating human genomic DNA Alu. Animals will be euthanized by $CO_2$ asphyxiation and cervical dislocation according to an IACUC approved protocol when tumor volume exceeds a diameter of 20 mm.

### Study 1

| Cohorts (n=3 NSG female mice per cohort) | 1 | 2 | 3 | 4 | 5 | 6 | 7 |
|---|---|---|---|---|---|---|---|
| MCF7 ($10^6$ cells) | + | + | + | + | + | + | + |
| hSVF ($10^6$ cells) (n = 3 pooled obese) | | | | + | + | + | + |
| Matrigel (25%) | | + | | + | | + | |
| ObaGel (25%) | | | + | | + | | + |
| Adipogenesis prior to implantation | | | | | | + | + |

### Study 2

| Cohorts (n = 3 NSG female mice per cohort) | 1 | 2 | 3 | 4 |
|---|---|---|---|---|
| MCF7, T47/D, or ZR75 ($10^6$ cells) | + | + | + | + |
| Obese SVF ($10^6$ cells) (pooled n = 3 donors) | + | | + | |
| Lean SVF ($10^6$ cells) (pooled n = 3 donors) | | + | | + |
| Matrigel (25%) | + | + | | |
| ObaGel (25%) | | | + | + |

Histology and Immunohistochemistry: Tumors will be fixed in 10% formalin, paraffin embedded, sectioned, and stained with hematoxylin & eosin (H&E) as previously described [17-19]. Tumor sections will be stained with antibodies detecting vascular (CD31), myeloid (CD11b or CD14), lymphoid (CD3, CD19), pericyte (CD146), or stromal (CD44 or CD73) surface antigens using an appropriate secondary antibody conjugated to alkaline phosphatase or horse radish peroxidase for detection. Image analysis will be performed with CellProfiler software (http://cellprofiler.org/).

SA1: Anticipated Outcomes:In Study 1, the inventors expect that the MCF7 cells will establish 20 mm diameter tumors no earlier than 5 weeks after implantation. While the addition of Matrigel alone is expected to accelerate the rate of MCF7 tumor growth, this will be matched or exceeded by Mammalian platelet lysate alone. The histology of these tumors will be relatively homogenous and will rely on murine host cells for any stromal or vascular network. In contrast, the presence of obese hSVF cells to either matrix will provide additional enhancement to the rate of tumor growth due to the presence of progenitor cells capable of providing a stromal and vascular network; the latter will be evident histologically with increased cell heterogeneity based on H&E and immunohistochemical staining. The maximal rate of growth will be obtained with the addition of hSVF cells and scaffolds pre-conditioned under adipogenic conditions. This is expected due to the increased production and secretion of leptin which has been implicated in the mechanism by which obesity contributes to breast cancer growth. In Study 2, the inventors expect that obese SVF cells will promote more rapid tumor growth for all breast cancer lines relative to lean SVF cells due to their increased secretion of leptin. Mammalian platelet lysate is expected to accelerate the cell lines growth equal to or greater than that observed with Matrigel.

Potential Pitfalls and Alternative Approaches: While prior studies were performed with culture expanded ASC, The inventors anticipates that SVF cells will perform as well or better than ASC due to their heterogeneity and inclusion of endothelial progenitors capable of vasculogenesis. Nevertheless, it is possible that SVF cells will not accelerate tumor cell growth as robustly as ASC. If this is observed in Study 1, alternative studies will be performed using a higher ratio of SVF cells relative to MCF7 cells (3.3 or 10:1 ratios). Additional controls substituting ASC for SVF cells will be added to the study design. Furthermore, the inventors anticipate that obese SVF cells will enhance tumor growth more robustly than lean SVF cells based on prior studies using ASC. Since this question is one that goes to the mechanism of the stromal/scaffold/tumor interaction, it goes beyond the initial "proof of principle" intent of this study. Consequently, the inventor will perform the Study 2 design comparing lean vs. obese hSVF cells using three different established tumor cell lines if the budget, timeline and personnel resources permit. This delay may prove to be advantageous since by that time, the inventors will have successfully developed derived PDX tumors "in house". The company's proprietary PDX model can then be evaluated in parallel with established tumor cell lines to examine the relative contributions of lean vs obese SVF cells to the PDX tumor growth rate.

Experiment 1 and Hypothesis 1 That Mammalian platelet lysate functions as well or better than Matrigel™ as in vivo scaffold for establishment of breast cancer patient derived xenografts implanted into immunodeficient mice (NSG) in combination with human SVF cells.

Rationale: While Matrigel is used routinely for in vitro cell culture of breast cancer cell lines, it is not routinely reported as a component during the establishment and maintenance of PDX models. Nevertheless, the colon cancer literature indicates that Matrigel improves and promotes PDX engraftment and subsequent tumor growth. While platelet lysates were first reported thirty years ago to improve colony formation from primary breast cancers, there have been few if any studies during the intervening years employing platelet lysates in breast cancer cell culture or PDX models. The inventor has developed novel proprietary products combining adipose-derived cells and human platelet lysates to create a "fat on a chip" model. Since adipose tissue and obesity promote breast cancer growth, the inventors will exploit its technology to accelerate and enhance breast cancer PDX growth in an immunodeficient murine model. By taking advantage of a commercialized human Alu based detection assay. The inventor will be able to effectively perform real time monitoring of breast cancer PDX engraftment and growth serially in individual mice without sacrificing the experimental animals. This advanced assay will reduce cost of PDX model production and accelerate its commercial development.

Experimental Design:

The authentication of biological reagents will be performed as outlined in Experiment 1. Additionally, the inventors will obtain tumor specimens from its collaborating hospital based biobank under an approved IRB protocol with either signed patient consent or where appropriate and allowed by the newly revised FDA regulations, with a waived signed consent. All specimens provided to the inventors will remain anonymous with respect to identifying metrics. The inventors will receive only that patient information relevant to the tumor and the patient demographics (age, gender, body mass index, ethnicity, past medical history, social history, tumor histology). Only the biobank and the designated physician investigator on its IRB protocol alone will have access to additional information relating to the tissue donors.

Methods - Technical methods will be performed as indicated under Experiment 1. Tumor specimens will be dissected under sterile technique into 3 mm3 fragments and implanted subcutaneously bilaterally on the flank of the anesthetized NSG mice. The tissue fragments will be implanted in the absence or presence of 250 μl of Matrigel or Mammalian platelet lysate and the absence or presence of 106 SVF cells. The scaffold plus SVF cells will have been injected without modification or following adipogenic induction in vitro prior to the implantation. An average of 12 mice will be implanted per tumor specimen donor (depending on tissue volume) with a minimum total of ten patients to be evaluated.

Experiment 2: Anticipated Outcomes: The inventors expect that the engraftment ('take') rate for the tumor alone will be ~20% in the absence of scaffold or adipose derived cells. Furthermore, the time to the physical appearance of the tumor will be longer when such tumors implanted without scaffold and/or SVF cells based on visual detection. The Alu based human genomic DNA detection system will prove to be more sensitive and will detect circulating human tumor cells at least 1 to 2 months in advance of physical and visual detection of the tumor growth. The presence of the Matrigel scaffold alone is expected to accelerate the rate of tumor (a) growth and (b) engraftment. The Mammalian platelet lysate alone is expected to be equal to or more effective than the Matrigel in accelerating the rates of tumor growth and increasing the rate of engraftment. Addition of the SVF cells will further accelerate tumor growth and engraftment rates above that of scaffolds alone. Moreover, the stromal network in the presence of SVF cells is expected to be more vascular and complex relative to scaffold alone or tumor alone. Finally, adipogenic differentiation of the scaffold plus SVF cells ("fat on a chip") will be expected to accelerate and enhance tumor growth above that of matrix and/or SVF cells alone or in combination without ex vivo adipogenic differentiation.

# EP 3 579 844 B1

**Claims**

1. A biological scaffold comprising

   - a mammalian platelet lysate, optionally from human platelets, and
   - stromal vascular fraction cells (SVF) comprising pre-adipocytes, vascular smooth muscle cells, endothelial cells and progenitors, B and T lymphocytes and progenitors, hematopoietic progenitors, macrophages, and monocytes,

   wherein said SVF cells are human cells.

2. The biological scaffold according to claim 1, wherein the SVF cells comprise endothelial progenitors, hematopoietic progenitors, and CD14-, CD36+ pre-adipocytes.

3. A therapeutic product comprising

   - a biological scaffold according to claim 1 and
   - a semi solid or solid substrate selected from a tissue culture plate, an orthopedic device, a dental implant, bone, a demineralized bone scaffold, a bandage, a metallic implant, a ceramic implant, a silk fibroin scaffold; and/or

   wherein the substrate comprises collagen, adipose extracellular matrix protein, a gel, a hPL gel, and wherein the substrate has at least one surface for application of the biological product by contacting said biological scaffold to the substrate by mixing, seeding, perfusing, loading by capillary action, loaded by centrifugation, injecting, printing, or bio-printing the biological scaffold to the at least one surface of the substrate.

4. The biological scaffold of claim 1, or the therapeutic product of claim 3, wherein the biological scaffold further comprises mesenchymal cells, neurons, neuronal progenitor cells, fibroblasts, dermal, epidermal, cancer-derived cells, cancer stem cells, chondrocytes, muscle cells, adipose cells, osteoblasts, osteoclasts, bone marrow cells, blood cells, and combinations thereof.

5. A method of making a biological scaffold containing product or device, the method comprising the following steps:

   - harvesting adipose tissue,
   - isolating SVF cells from the adipose tissue,
   - preparing a mammalian platelet lysate,
   - combining the SVF cells and the mammalian platelet lysate to form a biological scaffold, and
   - applying or contacting the biological scaffold to at least one surface of a substrate by mixing, seeding, perfusing, loading by capillary action, loaded by centrifugation, injecting, printing, or bio-printing and wherein the substrate is semi solid or solid substrate selected from an orthopedic device, a dental implant, bone, a demineralized bone scaffold, a synthetic mesh, a metallic implant, a ceramic implant.

6. The method of claim 5, wherein the SVF cells comprise pre-adipocytes, vascular smooth muscle cells, endothelial cells and progenitors, B and T lymphocytes and progenitors, hematopoietic progenitors, macrophages, and monocytes.

7. The method of claim 5, wherein the SVF cells comprise endothelial progenitors, hematopoietic progenitors, and CD14-, CD36+ pre-adipocytes.

8. A method of testing the efficacy or toxicity of a pharmacological agent, the method comprising:

   - preparing a biological scaffold comprising a mammalian platelet lysate, optionally from human platelets, and SVF cells comprising pre-adipocytes, vascular smooth muscle cells, endothelial cells and progenitors, B and T lymphocytes and progenitors, hematopoietic progenitors, macrophages, monocytes, and wherein the SVF cells are human cells,
   - contacting the biological scaffold with a pharmacological agent,
   - culturing the biological scaffold with the pharmacological agent, and
   - testing the viability of cells within the biological scaffold.

9. The method according to claim 8, wherein the pharmacological agent comprises: growth factors, differentiation factors, cytokines, chemokines, adipokines, antibiotics, antimycotics, anti-fungals, anti-inflammatories, or anti-cancer agents.

10. An immunocompromised or immunodeficient mouse comprising

- a functioning human fat pad comprising a biological scaffold and
- a replicate pathology of a human disease, which is a tumor cell line or tumor fragment,
wherein the mouse is a xenografted mouse,
wherein the biological scaffold comprises a mammalian platelet lysate, optionally from human platelets, and SVF cells comprising pre-adipocytes, vascular smooth muscle cells, endothelial cells and progenitors, B and T lymphocytes and progenitors, hematopoietic progenitors, macrophages, monocytes and
wherein the SVF cells are human cells.

11. A microfluidic apparatus containing one or more biological scaffolds, each biological scaffold comprising a mammalian platelet lysate, optionally from human platelets, and SVF cells, the SFV cells comprising pre-adipocytes, vascular smooth muscle cells, endothelial cells and progenitors, B and T lymphocytes and progenitors, hematopoietic progenitors, macrophages, and monocytes,

wherein the SVF cells are human cells,
wherein the biological scaffolds are isolated and can be subjected to uniform flow of culture media and/or pharmacological agents.

12. A method of testing the efficacy or toxicity of a pharmacological agent, the method comprising:

- preparing at least two biological scaffolds, each biological scaffold comprising a mammalian platelet lysate, optionally from human platelets, and SVF cells comprising pre-adipocytes, vascular smooth muscle cells, endothelial cells and progenitors, B and T lymphocytes and progenitors, hematopoietic progenitors, macrophages, and monocytes, and

wherein the SVF cells are human cells and
wherein the at least two biological scaffolds are isolated from each other and contained within a microfluidic apparatus,

- treating the microfluidic apparatus with a pharmacological agent,
- culturing the microfluidic apparatus with the pharmacological agent, and
- testing the viability of cells within the microfluidic apparatus

**Patentansprüche**

1. Ein biologisches Gerüst, welches:

- ein Säugetier-Blutplättchenlysat, optional aus humanen Blutplättchen, und
- stromal-vaskuläre Fraktionszellen (SVF), die Präadipocyten, glatte Gefäßmuskelzellen, Endothelzellen und deren Progenitorzellen, B- und T-Lymphocyten und deren Progenitorzellen, hämatopoetische Progenitorzellen, Makrophagen und Monocyten umfassen,

umfasst, wobei die SVF-Zellen humane Zellen sind.

2. Das biologische Gerüst nach Anspruch 1, wobei die SVF-Zellen Endothelprogenitorzellen, hämatopoetische Progenitorzellen und CD14--, CD36+-Präadipocyten umfassen.

3. Ein therapeutisches Erzeugnis, welches:

- ein biologisches Gerüst nach Anspruch 1 und
- ein halbfestes oder festes Substrat umfasst, das aus einer Gewebekulturplatte, einer orthopädischen Vorrichtung, einem Zahnimplantat, einem Knochen, einem entmineralisierten Knochengerüst, einer Bandage, einem Metallimplantat, einem Keramikimplantat und einem Seidenfibroingerüst ausgewählt ist, und/oder

wobei das Substrat Collagen, extrazelluläres Fettmatrixprotein, ein Gel und ein hPL-Gel umfasst, und wobei das Substrat mindestens eine Fläche für das Aufbringen des biologischen Erzeugnisses durch In-Berührung-Bringen des biologischen Gerüsts mit dem Substrat durch Mischen, Aussäen, Perfundieren, Beladen durch Kapillarwirkung, Beladen durch Zentrifugieren, Injizieren, Drucken oder Biodrucken des biologischen Gerüsts auf die/der mindestens eine/n Oberfläche des Substrats besitzt.

4.  Das biologische Gerüst nach Anspruch 1 oder das therapeutische Erzeugnis nach Anspruch 3, wobei das biologische Gerüst ferner Mesenchymzellen, Neuronen, neuronale Progenitorzellen, Fibroblasten, Dermis-, Epidermis und von Krebs abgeleitete Zellen, Krebsstammzellen, Chondrocyten, Muskelzellen, Fettzellen, Osteoblasten, Osteoklasten, Knochenmarkszellen, Blutzellen und Kombinationen davon umfasst.

5.  Ein Verfahren zur Herstellung eines/einer ein biologisches Gerüst enthaltenden Erzeugnisses oder Vorrichtung, wobei das Verfahren die Schritte:

    - Ernten eines Fettgewebes,
    - Trennen der SVF-Zellen von dem Fettgewebe,
    - Herstellen eines Säugetier-Blutplättchenlysats
    - Vereinigen der SVF-Zellen mit dem Säugetier-Blutplättchenlysat, um ein biologisches Gerüst zu bilden, und
    - Aufbringen oder In-Berührung-Bringen des biologischen Gerüsts auf/mit mindestens eine/r Fläche eines Substrats durch Mischen, Aussäen, Perfundieren, Beladen durch Kapillarwirkung, Beladen durch Zentrifugieren, Injizieren, Drucken oder Biodrucken und wobei das Substrat ein halbfestes oder festes Substrat ist, das aus einer orthopädischen Vorrichtung, einem Zahnimplantat, einem Knochen, einem entmineralisierten Knochengerüst, einem synthetischen Netz, einem Metallimplantat und einem Keramikimplantat ausgewählt ist,

    umfasst.

6.  Das Verfahren nach Anspruch 5, wobei die SVF-Zellen Präadipocyten, glatte Gefäßmuskelzellen, Endothelzellen und deren Progenitorzellen, B- und T-Lymphocyten und deren Progenitorzellen, hämatopoetische Progenitorzellen, Makrophagen und Monocyten umfassen.

7.  Das Verfahren nach Anspruch 5, wobei die SVF-Zellen Endothelprogenitorzellen, hämatopoetische Progenitorzellen und CD14--, CD36+-Präadipocyten umfassen.

8.  Ein Verfahren zum Testen der Wirksamkeit oder der Toxizität eines pharmakologischen Agens, wobei das Verfahren:

    - Herstellen eines biologischen Gerüsts, das ein Säugetier-Blutplättchenlysat, optional aus humanen Blutplättchen, und SVF-Zellen, die Präadipocyten, glatte Gefäßmuskelzellen, Endothelzellen und deren Progenitorzellen, B- und T-Lymphocyten und deren Progenitorzellen, hämatopoetische Progenitorzellen, Makrophagen und Monocyten umfassen, umfasst, und wobei die SVF-Zellen humane Zellen sind,
    - In-Berührung-Bringen des biologischen Gerüsts mit einem pharmakologischen Agens,
    - Kultivieren des biologischen Gerüsts mit dem pharmakologischen Agens und
    - Testen der Lebensfähigkeit der Zellen in dem biologischen Gerüst

    umfasst.

9.  Das Verfahren nach Anspruch 8, wobei das pharmakologische Agens Wachstumsfaktoren, Differenzierungsfaktoren, Cytokine, Chemokine, Adipokine, Antibiotika, Antimykotika, Antipilzmittel, Entzündungshemmer oder Antikrebsmittel umfasst.

10. Eine immungeschwächte oder immundefiziente Maus, welche:

    - eine ein biologisches Gerüst umfassende funktionierende humane Fettschicht und
    - eine reproduzierte Pathologie einer humanen Krankheit, die eine Tumorzelllinie oder ein Tumorfragment ist, umfasst,

    wobei die Maus eine xenotransplantierte Maus ist, wobei das biologische Gerüst ein Säugetier-Blutplättchenlysat, optional aus humanen Blutplättchen, und SVF-Zellen, die Präadipocyten, glatte Gefäßmuskelzellen, Endothelzellen und deren Progenitorzellen, B- und T-Lymphocyten und deren Progenitorzellen, hämatopoetische Progenitorzellen, Makrophagen und Monocyten umfassen, umfasst, wobei die SVF-Zellen humane Zellen sind.

11. Eine mikrofluidische Vorrichtung, die ein oder mehrere biologische Gerüste enthält, wobei jedes biologische Gerüst ein Säugetier-Blutplättchenlysat, optional aus humanen Blutplättchen, und SVF-Zellen, die Präadipocyten, glatte Gefäßmuskelzellen, Endothelzellen und deren Progenitorzellen, B- und T-Lymphocyten und deren Progenitorzellen, hämatopoetische Progenitorzellen, Makrophagen und Monocyten umfassen, umfasst,

wobei die SVF-Zellen humane Zellen sind,
wobei die biologischen Gerüste isoliert sind und einem gleichmäßigen Durchfluss von Kulturmedien und/oder pharmakologischen Agenzien unterworfen werden können.

12. Ein Verfahren zum Testen der Wirksamkeit oder Toxizität eines pharmakologischen Agens, wobei das Verfahren das:

- Herstellen mindestens zweier biologischer Gerüste, wobei jedes biologische Gerüst ein Säugetier-Blutplättchenlysat, optional von humanen Blutplättchen, und SVF-Zellen, die Präadipocyten, glatte Gefäßmuskelzellen, Endothelzellen und deren Progenitorzellen, B- und T-Lymphocyten und deren Progenitorzellen, hämatopoetische Progenitorzellen, Makrophagen und Monocyten umfassen, umfasst, und wobei die SVF-Zellen humane Zellen sind und wobei die mindestens zwei biologischen Gerüste isoliert voneinander und in einer mikrofluidischen Vorrichtung enthalten sind,
- Behandeln der mikrofluidischen Vorrichtung mit einem pharmakologischen Agens,
- Kultivieren der mikrofluidischen Vorrichtung mit dem pharmakologischen Agens und
- Testen der Lebensfähigkeit der Zellen in der mikrofluidischen Vorrichtung

umfasst.

## Revendications

1. Échafaudage biologique comprenant

- un lysat plaquettaire de mammifère, éventuellement provenant de plaquettes humaines, et
- des cellules de fraction vasculaire stromale (SVF) comprenant préadipocytes, cellules musculaires lisses vasculaires, cellules endothéliales et progéniteurs endothéliaux, lymphocytes B et T et progéniteurs B et T, progéniteurs hématopoïétiques, macrophages et monocytes,

dans lequel lesdites cellules SVF sont des cellules humaines.

2. Échafaudage biologique selon la revendication 1, dans lequel les cellules SVF comprennent progéniteurs endothéliaux, progéniteurs hématopoïétiques et préadipocytes CD14-, CD36+.

3. Produit thérapeutique comprenant

- un échafaudage biologique selon la revendication 1 et
- un substrat semi-solide ou solide choisi parmi plaque de culture tissulaire, dispositif orthopédique, implant dentaire, os, échafaudage osseux déminéralisé, bandage, implant métallique, implant en céramique, échafaudage de fibroïne de soie ; et/ou

dans lequel le substrat comprend collagène, protéine de matrice extracellulaire adipeuse, gel, gel hPL, et dans lequel le substrat a au moins une surface pour l'application du produit biologique en mettant en contact ledit échafaudage biologique avec le substrat par mélange, ensemencement, perfusion, chargement par capillarité, chargé par centrifugation, injection, impression ou bio-impression de l'échafaudage biologique sur l'au moins une surface du substrat.

4. Échafaudage biologique selon la revendication 1, ou produit thérapeutique selon la revendication 3, dans lequel l'échafaudage biologique comprend en outre cellules mésenchymateuses, neurones, cellules progénitrices neurales, fibroblastes, cellules dermiques, cellules épidermiques, cellules dérivées du cancer, cellules souches cancéreuses, chondrocytes, cellules musculaires, cellules adipeuses, ostéoblastes, ostéoclastes, cellules de moelle osseuse, cellules sanguines, et des combinaisons de ceux-ci.

5. Procédé pour la fabrication d'un produit ou d'un dispositif contenant un échafaudage biologique, le procédé comprenant les étapes suivantes :

- prélèvement de tissu adipeux,
- isolement de cellules SVF du tissu adipeux,
- préparation d'un lysat plaquettaire de mammifère,
- combinaison des cellules SVF et du lysat plaquettaire de mammifère pour former un échafaudage biologique, et
- application de l'échafaudage biologique sur au moins une surface d'un substrat ou mise en contact avec celle-ci par mélange, ensemencement, perfusion, chargement par capillarité, chargé par centrifugation, injection, impression ou bio-impression, et dans lequel le substrat est un substrat semi-solide ou solide choisi parmi dispositif orthopédique, implant dentaire, os, échafaudage osseux déminéralisé, maille synthétique, implant métallique, implant en céramique.

6. Procédé selon la revendication 5, dans lequel les cellules SVF comprennent préadipocytes, cellules musculaires lisses vasculaires, cellules endothéliales et progéniteurs endothéliaux, lymphocytes B et T et progéniteurs B et T, progéniteurs hématopoïétiques, macrophages et monocytes.

7. Procédé selon la revendication 5, dans lequel les cellules SVF comprennent progéniteurs endothéliaux, progéniteurs hématopoïétiques et préadipocytes CD14-, CD36+.

8. Procédé permettant de tester l'efficacité ou la toxicité d'un agent pharmacologique, le procédé comprenant :

- la préparation d'un échafaudage biologique comprenant un lysat plaquettaire de mammifère, éventuellement provenant de plaquettes humaines, et des cellules SVF comprenant préadipocytes, cellules musculaires lisses vasculaires, cellules endothéliales et progéniteurs endothéliaux, lymphocytes B et T et progéniteurs B et T, progéniteurs hématopoïétiques, macrophages, monocytes, et dans lequel les cellules SVF sont des cellules humaines,
- la mise en contact de l'échafaudage biologique avec un agent pharmacologique,
- la mise en culture de l'échafaudage biologique avec l'agent pharmacologique, et
- le test de la viabilité des cellules au sein de l'échafaudage biologique.

9. Procédé selon la revendication 8, dans lequel l'agent pharmacologique comprend : facteurs de croissance, facteurs de différenciation, cytokines, chimiokines, adipokines, antibiotiques, antimycosiques, antifongiques, anti-inflammatoires ou agents anticancéreux.

10. Souris immunodéprimée ou immunodéficiente comprenant

- un coussinet adipeux humain fonctionnel comprenant un échafaudage biologique et
- une pathologie répliquée d'une maladie humaine, qui est une lignée cellulaire de tumeur ou un fragment de tumeur,

dans laquelle la souris est une souris à xénogreffe,
dans laquelle l'échafaudage biologique comprend un lysat plaquettaire de mammifère, éventuellement provenant de plaquettes humaines, et des cellules SVF comprenant préadipocytes, cellules musculaires lisses vasculaires, cellules endothéliales et progéniteurs endothéliaux, lymphocytes B et T et progéniteurs B et T, progéniteurs hématopoïétiques, macrophages, monocytes et
dans laquelle les cellules SVF sont des cellules humaines.

11. Appareil microfluidique contenant un ou plusieurs échafaudages biologiques, chaque échafaudage biologique comprenant un lysat plaquettaire de mammifère, éventuellement provenant de plaquettes humaines, et des cellules SVF, les cellules SFV comprenant préadipocytes, cellules musculaires lisses vasculaires, cellules endothéliales et progéniteurs endothéliaux, lymphocytes B et T et progéniteurs B et T, progéniteurs hématopoïétiques, macrophages et monocytes,

dans lequel les cellules SVF sont des cellules humaines,

dans lequel les échafaudages biologiques sont isolés et peuvent être soumis à un flux uniforme de milieux de culture et/ou d'agents pharmacologiques.

12. Procédé permettant de tester l'efficacité ou la toxicité d'un agent pharmacologique, le procédé comprenant :

- la préparation d'au moins deux échafaudages biologiques, chaque échafaudage biologique comprenant un lysat plaquettaire de mammifère, éventuellement provenant de plaquettes humaines, et des cellules SVF comprenant préadipocytes, cellules musculaires lisses vasculaires, cellules endothéliales et progéniteurs endothéliaux, lymphocytes B et T et progéniteurs B et T, progéniteurs hématopoïétiques, macrophages et monocytes, et

dans lequel les cellules SVF sont des cellules humaines et
dans lequel les au moins deux échafaudages biologiques sont isolés l'un de l'autre et contenus dans un appareil microfluidique,

- le traitement de l'appareil microfluidique avec un agent pharmacologique,
- la mise en culture de l'appareil microfluidique avec l'agent pharmacologique, et
- le test de la viabilité des cellules au sein de l'appareil microfluidique.

Figure 1

A. Subpopulations in GFP-Tg male iWAT-SVF

B.

C.

Figure 2

Figure 3

Figure 4

Figure 4 (continued)

SVF T₂ immunophenotype

Figure 5

Figure 6

Figure 7

Figure 8

Figure 9

A.  DAPI          GFP          C.  Differentiation

B.  Bright Field     Merge        D.  Cryo-SEM

E.

Figure 10

Figure 11

Figure 12

EP 3 579 844 B1

Figure 13

SVF/hPL Gel stability

Correlation between Gel Effect and Cell Number

48

Figure 14

Figure 15

Figure 16

Figure 17

Lipolysis in 2D versus 3D

2D v 3D WAT v BAT

Figure 18

hPL control

hPL + cells

silk control

silk + cells

**EP 3 579 844 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62457366
- US 2018017847 W
- EP 2540819 A1
- WO 2014145753 A1

**Non-patent literature cited in the description**

- **THEODORA et al.** *Journal of Applied Polymer Sciences*, 05 February 2016, vol. 133 (5)